# EUROPEAN PATENT APPLICATION

(11) **EP 2 116 303 A1**
(43) Date of publication of application: **11.11.2009**
(21) Application number: 08720304.8
(22) Date of filing: 28.02.2008
(51) Int. Cl.: B01J 31/26, C07B 41/06, C07B 41/08, C07C 45/36, C07C 47/542, C07C 51/265, C07C 63/04, C07C 63/26, C07B 61/00, C07D 209/48

(54) **IMMOBILIZED CYCLIC IMIDE CATALYST AND PROCESS FOR OXIDATION OF ORGANIC COMPOUNDS WITH THE SAME**

(30) Priority: 08.03.2007 JP 2007059081
(71) Applicant: Daicel Chemical Industries, Ltd., Kita-ku, Osaka-shi Osaka 530-0001 (JP)
(72) Inventor: ISHII, Yasutaka, Takatsuki-shi Osaka 569-1112 (JP); TAKANO, Minoru, Himeji-shi, Hyogo 671-1283 (JP); HIRAI, Naruhisa, Himeji-shi, Hyogo 671-1283 (JP)
(74) Representative: Grünecker, Kinkeldey, Stockmair & Schwanhäusser Anwaltssozietät
(86) International application number: PCT/JP2008/000390
(87) International publication number: WO 2008/108073

(57) **Abstract**

To provide a solid catalyst containing a cyclic imide skeleton which is easily available and easily separable from reaction products and which is easily recovered and regenerated, and free from reaction inhibitory factors; and a process for oxidation of organic compounds with the solid catalyst.

An immobilized cyclic imide catalyst having a structure represented by following Formula (1), wherein X is oxygen or an -OR group (wherein R is hydrogen atom or a hydroxyl-protecting group); n is 0 or 1; Z¹ is a five- or six-membered cyclic imide skeleton to which an aromatic or nonaromatic ring Z² may be adjacent; elliptically shaped moiety S is an inorganic support; A¹ is a group linking silicon atom with the cyclic imide skeleton Z¹ or with the ring Z² and is either a divalent hydrocarbon group or a group composed of a divalent hydrocarbon group and an amide bond (-NHCO-); and the cyclic imide skeleton Z¹ and the ring Z² may each be substituted.

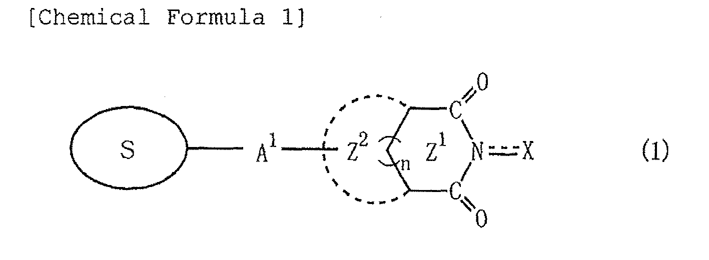

## Description

### Technical Field

The present invention relates to immobilized cyclic imide catalysts that are useful for oxidation reactions; and to processes for the oxidation of organic compounds using the catalysts.

### Background Art

An oxidation reaction is one of the most basic reactions in organic chemical industry, and there have been developed a variety of oxidation processes. Preferred oxidation processes from the viewpoints of resource and environment are catalytic oxidation process in which molecular oxygen or air is directly used as an oxidizing agent. The catalytic oxidation processes, however, generally require a high temperature and/or a high pressure for activating oxygen, or require a reaction in the coexistence of a reducing agent such as an aldehyde in order to carry out the reaction under mild conditions. It is therefore difficult for the catalytic oxidation processes to produce alcohols and carboxylic acids efficiently in a simple manner under mild conditions

Japanese Unexamined Patent Application Publication (JP-A) No. H08-38909 and JP-A No. H09-327626 propose oxidation catalysts each including an imide compound having a specific structure, such as N-hydroxyphthalimide, alone or in combination typically with a transition metallic compound, as catalysts for the oxidation of organic substrates with molecular oxygen. According to these processes using imide compounds as catalysts, the oxidation of organic compounds such as hydrocarbons can be performed under relatively mild conditions to give oxidized products such as hydroperoxides, alcohols, carbonyl compounds, and carboxylic acids in relatively high yields. These processes, however, are all based on liquid-phase homogeneous reactions and thereby have a need of complicated procedures to separate and recover a target compound and the catalyst from a reaction mixture.

JP-A No. 2002-282698 discloses an N-hydroxyphthalimide catalyst bound to a solid through an aminoalkyl group, as an N-hydroxyphthalimide catalyst that can be isolated and recovered from a reaction solution after the completion of the reaction without the need of complicated separation procedures. This catalyst, however, is disadvantageous in cost and production facilities, because the catalyst uses 4-formyl-N-hydroxyphthalimide as a raw material and inevitably uses sodium borohydride as a reducing agent, but 4-formyl-N-hydroxyphthalimide is hardly available and sodium borohydride is a water prohibitive substance and is thereby difficult to deal with. Additionally, the catalyst is not desirable in consideration typically of the long-term use of catalyst, because it is an amine compound and may act as a free-radical inhibitor by the action of an unshared electron pair on the amine nitrogen atom to thereby inhibit the oxidation reaction.

Patent Document 1: JP-A No. H08-38909
Patent Document 2: JP-A No. H09-327626
Patent Document 3: JP-A No. 2002-282698

### Disclosure of Invention

### Problems to be Solved by the Invention

Accordingly, an object of the present invention is to provide an immobilized cyclic imide catalyst that is easily available, can be easily separated from a reaction product, can be recovered and regenerated in a simple manner, and has no inhibitory factor being inhibitory upon a target reaction. Another object of the present invention is to provide a process for the oxidation of organic compounds using the immobilized catalyst.

### Means for Solving the Problems

After intensive investigations to achieve the objects, the present inventors have found a solid catalyst (catalyst including an immobilized cyclic imide skeleton) composed of an inorganic support and a cyclic imide skeleton bound to each other through a specific linkage group (spacer) and have found that this solid catalyst, if used, enables smooth oxidation of organic compounds under mild conditions; can be easily separated and recovered from reaction products; can be easily prepared; includes no inhibitory factor upon the oxidation reaction, can thereby be used over a long period of time; if recovered, can be regenerated in a simple manner, and is reusable in reactions. The present invention has been made based on these findings.

Specifically, the present invention provides an immobilized cyclic imide catalyst which has a structure represented by following Formula (1): 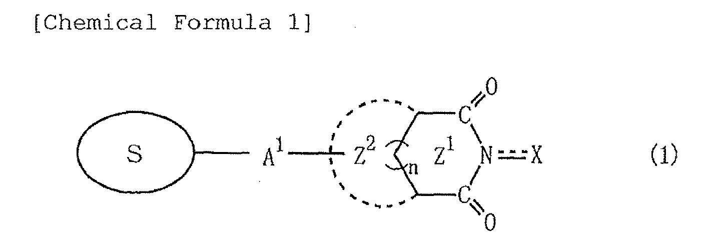 wherein X represents oxygen atom or an -OR group, wherein R represents hydrogen atom or a hydroxyl-protecting group; "n" represents 0 or 1; Z¹ represents a five- or six-membered cyclic imide skeleton to which an aromatic or nonaromatic ring Z² may be adjacent; elliptically shaped moiety "S" represents an inorganic support; A¹ represents a linkage group linking the inorganic support S with the cyclic imide skeleton Z¹ or with the ring Z² and is either a bivalent hydrocarbon group or a group composed of a bivalent hydrocarbon group and an amide bond (-NHCO-); the cyclic imide skeleton Z¹ and the ring Z² may each be substituted; and the structure may have, per molecule, two or more of the cyclic imide skeleton Z¹ to which the ring Z² may be adjacent.

In Formula (1), it is preferred that (i) "n" is 0 and the ring Z² is a six-membered aromatic or nonaromatic carbon ring possessing one side in common with the cyclic imide skeleton Z¹, or that (ii) "n" is 1 and the ring Z² is naphthalene ring or decahydronaphthalene ring possessing two sides in common with the cyclic imide skeleton Z¹.

Examples of the inorganic support S include at least one member selected from the group consisting of a silica, an alumina, a titania, a zirconia and a ceria; and a composite oxide of two or more elements selected from the group consisting of silicon, aluminum, zirconium and cerium.

The present invention further provides a process for the oxidation of an organic compound. The process includes the step of carrying out an oxidation reaction of the organic compound in the presence of the immobilized cyclic imide catalyst.

The oxidation reaction in the oxidation process may be performed in a fluidized bed system or fixed bed system. According to the oxidation process, a hydrocarbon can be oxidized into at least one compound selected from the group consisting of a hydroperoxide, an alcohol, a carbonyl compound and a carboxylic acid.

In Formula (1), a group present at the bonding site between the inorganic support S and the linkage group A¹ is defined as belonging to the inorganic support S (elliptically shaped moiety "S"). Exemplary groups at the bonding site include groups, such as a siloxane bond, which are formed when a surface functional group of the inorganic support S is bound to a terminal functional group of the linkage group A¹ typically through a silane coupling reaction.

### Advantages

The present invention enables smooth oxidation of organic compounds such as hydrocarbons under mild conditions. Catalysts used herein, as being solid catalysts, can be easily separated from reaction products and can be easily recovered from a reaction mixture. The catalysts can also be easily prepared, because the linkage group linking the cyclic imide skeleton with the inorganic support is a bivalent hydrocarbon group or a group composed of a bivalent hydrocarbon group and an amide bond. In addition, the catalysts are suitable as catalysts for oxidation reactions and can be used over a long period of time, because the linkage group has no inhibitory factor upon oxidation reactions. The recovered catalysts, if deactivated, can be regenerated according to a simple procedure, and the regenerated catalysts are reusable.

### Best Modes for Carrying Out the Invention

### [Solid Catalyst Containing Cyclic Imide Skeleton]

Immobilized cyclic imide catalysts according to the present invention each have a structure represented by Formula (1). In Formula (1), the bond between the nitrogen atom and X is either a single bond or a double bond. X represents oxygen atom or an -OR group, wherein R represents hydrogen atom or a hydroxyl-protecting group; "n" represents 0 or 1; and Z¹ represents a five- or six-membered cyclic imide skeleton to which an aromatic or nonaromatic ring Z² may be adjacent. The structure may have, per molecule, two or more of the cyclic imide skeleton Z¹ to which the ring Z² may be adjacent.

When X is an -OR group and R is a hydroxyl-protecting group in Formula (1), two or more of moieties of the solid catalyst represented by Formula (1) other than R may be bound to each other through R.

The hydroxyl-protecting group as R in Formula (1) can be a hydroxyl-protecting group commonly used in organic synthesis. Exemplary protecting groups herein include alkyl groups (e.g., alkyl groups having 1 to 4 carbon atoms, such as methyl and t-butyl groups), alkenyl groups (e.g., allyl group), cycloalkyl groups (e.g., cyclohexyl group), aryl groups (e.g., 2,4-dinitrophenyl group), and aralkyl groups (e.g., benzyl, 2,6-dichlorobenzyl, 3-bromobenzyl, 2-nitrobenzyl, and triphenylmethyl groups); groups capable of forming an acetal or hemiacetal group with hydroxyl group, including substituted methyl groups (e.g., methoxymethyl, methylthiomethyl, benzyloxymethyl, t-butoxymethyl, 2-methoxyethoxymethyl, 2,2,2-trichloroethoxymethyl, bis(2-chloroethoxy)methyl, and 2-(trimethylsilyl)ethoxymethyl groups), substituted ethyl groups (e.g., 1-ethoxyethyl, 1-methyl-1-methoxyethyl, 1-isopropoxyethyl, 2,2,2-trichloroethyl, and 2-methoxyethyl groups), tetrahydropyranyl group, tetrahydrofuranyl group, and 1-hydroxyalkyl groups (e.g., 1-hydroxyethyl, 1-hydroxyhexyl, 1-hydroxydecyl, 1-hydroxyhexadecyl, and 1-hydroxy-1-phenylmethyl groups); acyl groups (e.g., aliphatic saturated or unsaturated acyl groups including aliphatic acyl groups having 1 to 20 carbon atoms, such as formyl, acetyl, propionyl, butyryl, isobutyryl, valeryl, pivaloyl, hexanoyl, heptanoyl, octanoyl, nonanoyl, decanoyl, lauroyl, myristoyl, palmitoyl, and stearoyl groups; acetoacetyl group; alicyclic acyl groups including cycloalkanecarbonyl groups such as cyclopentanecarbonyl and cyclohexanecarbonyl groups; and aromatic acyl groups such as benzoyl and naphthoyl groups), sulfonyl groups (e.g., methanesulfonyl, ethanesulfonyl, trifluoromethanesulfonyl, benzenesulfonyl, p-toluenesulfonyl, and naphthalenesulfonyl groups), alkoxycarbonyl groups (e.g., alkoxy-carbonyl groups whose alkoxy moiety having 1 to 4 carbon atoms, such as methoxycarbonyl, ethoxycarbonyl, and t-butoxycarbonyl groups), aralkyloxycarbonyl groups (e.g., benzyloxycarbonyl group and p-methoxybenzyloxycarbonyl group), substituted or unsubstituted carbamoyl groups (e.g., carbamoyl, methylcarbamoyl, and phenylcarbamoyl groups), groups corresponding to inorganic acids (e.g., sulfuric acid, nitric acid, phosphoric acid, and boric acid) except for removing hydroxyl group (OH group) therefrom, dialkylphosphinothioyl groups (e.g., dimethylphosphinothioyl group), diarylphosphinothioyl groups (e.g., diphenylphosphinothioyl group), and substituted silyl groups (e.g., trimethylsilyl, t-butyldimethylsilyl, tribenzylsilyl, and triphenylsilyl group).

When X is an -OR group and two or more moieties of the solid catalyst represented by Formula (1) other than R are bound to each other through R, exemplary Rs include acyl groups of polycarboxylic acids, such as oxalyl, malonyl, succinyl, glutaryl, phthaloyl, isophthaloyl, and terephthaloyl groups; carbonyl group; and multivalent hydrocarbon groups such as methylene, ethylidene, isopropylidene, cyclopentylidene, cyclohexylidene, and benzylidene groups, of which groups that form acetal bonds with two hydroxyl groups are preferred.

Preferred examples of R include hydrogen atom; groups capable of forming an acetal or hemiacetal group with hydroxyl group; groups corresponding to acids, such as carboxylic acids, sulfonic acids, carbonic acid, carbamic acid, sulfuric acid, phosphoric acid, and boric acid, except for removing hydroxyl group therefrom (acyl groups, sulfonyl groups, alkoxycarbonyl groups, and carbamoyl groups), and other hydrolyzable protecting groups capable of being removed (deprotected) through hydrolysis. R is especially preferably hydrogen atom.

Examples of the aromatic or nonaromatic ring Z² include rings having about 5 to about 12 members, of which rings having about 6 to about 10 members are preferred. The ring Z² may be a heterocyclic ring or fused heterocyclic ring, but it is often a hydrocarbon ring. Examples of such rings include alicyclic rings (e.g., cycloalkane rings such as cyclohexane ring, and cycloalkene rings such as cyclohexene ring), bridged rings (e.g., bridged hydrocarbon rings such as 5-norbornene ring), and aromatic rings (including fused rings) such as benzene ring and naphthalene ring. The ring is often composed of an aromatic ring.

The cyclic imide skeleton Z¹ and the ring Z² may each be substituted. Exemplary substituents on the cyclic imide skeleton Z¹ include halogen atoms, alkyl groups, aryl groups, cycloalkyl groups, hydroxyl group, alkoxy groups, carboxyl group, substituted oxycarbonyl groups, acyl groups, and acyloxy groups.

The halogen atoms include iodine, bromine, chlorine, and fluorine atoms. Examples of the alkyl groups include linear or branched-chain alkyl groups having 1 to 30 carbon atoms, such as methyl, ethyl, propyl, isopropyl, butyl, hexyl, decyl, and dodecyl groups, of which those having about 1 to about 20 carbon atoms are preferred.

The aryl groups include, for example, phenyl, tolyl, xylyl, and naphthyl groups; and the cycloalkyl groups include, for example, cyclopentyl and cyclohexyl groups. Examples of the alkoxy groups include alkoxy groups having 1 to 30 carbon atoms, such as methoxy, ethoxy, isopropoxy, butoxy, t-butoxy, hexyloxy, decyloxy, and dodecyloxy groups, of which those having about 1 to about 20 carbon atoms are preferred.

Examples of the substituted oxycarbonyl groups include alkoxy-carbonyl groups whose alkoxy moiety having 1 to 30 carbon atoms, such as methoxycarbonyl, ethoxycarbonyl, isopropoxycarbonyl, butoxycarbonyl, t-butoxycarbonyl, hexyloxycarbonyl, and decyloxycarbonyl groups, of which alkoxy-carbonyl groups whose alkoxy moiety having 1 to 20 carbon atoms are preferred; cycloalkyloxycarbonyl groups such as cyclopentyloxycarbonyl and cyclohexyloxycarbonyl groups, of which cycloalkyloxycarbonyl groups having 3 to 20 members are preferred; aryloxycarbonyl groups such as phenyloxycarbonyl group, of which aryloxy-carbonyl groups whose aryloxy moiety having 6 to 20 carbon atoms are preferred; and aralkyloxycarbonyl groups such as benzyloxycarbonyl group, of which aralkyloxy-carbonyl groups whose aralkyloxy moiety having 7 to 21 carbon atoms are preferred.

The acyl groups include, for example, aliphatic saturated or unsaturated acyl groups including aliphatic acyl groups having 1 to 30 carbon atoms, such as formyl, acetyl, propionyl, butyryl, isobutyryl, valeryl, pivaloyl, hexanoyl, decanoyl, and lauroyl groups, of which aliphatic acyl groups having 1 to 20 carbon atoms are preferred; acetoacetyl group; alicyclic acyl groups including cycloalkanecarbonyl groups such as cyclopentanecarbonyl and cyclohexanecarbonyl group; and aromatic acyl groups such as benzoyl group.

Examples of the acyloxy groups include aliphatic saturated or unsaturated acyloxy groups including aliphatic acyloxy groups having 1 to 30 carbon atoms, such as formyloxy, acetyloxy, propionyloxy, butyryloxy, isobutyryloxy, valeryloxy, pivaloyloxy, decanoyloxy, and lauroyloxy groups, of which aliphatic acyloxy groups having 1 to 20 carbon atoms are preferred; acetoacetyloxy group; alicyclic acyloxy groups including cycloalkanecarbonyloxy groups such as cyclopentanecarbonyloxy and cyclohexanecarbonyloxy groups; and aromatic acyloxy groups such as benzoyloxy group.

Exemplary substituents on the ring Z² include alkyl groups, haloalkyl groups, hydroxyl group, alkoxy groups, carboxyl group, substituted oxycarbonyl groups, acyl groups, acyloxy groups, nitro group, cyano group, amino groups, and halogen atoms. The alkyl groups include alkyl groups as with the alkyl groups exemplified as the substituents on the cyclic imide skeleton Z¹, of which alkyl groups having about 1 to about 6 carbon atoms are preferred. The haloalkyl groups include haloalkyl groups having about 1 to about 10 carbon atoms, such as trifluoromethyl group, of which those having about 1 to about 4 carbon atoms are preferred. The alkoxy groups include alkoxy groups as above, of which lower alkoxy groups having about 1 to about 4 carbon atoms are preferred. The substituted oxycarbonyl groups include substituted oxycarbonyl groups as above, such as alkoxycarbonyl groups, cycloalkyloxycarbonyl groups, aryloxycarbonyl groups, and aralkyloxycarbonyl groups. Examples of the acyl groups include acyl groups as above, such as aliphatic saturated or unsaturated acyl groups, acetoacetyl groups, alicyclic acyl groups, and aromatic acyl groups. Examples of the acyloxy groups include acyloxy groups as above, such as aliphatic saturated or unsaturated acyloxy groups, acetoacetyloxy group, alicyclic acyloxy groups, and aromatic acyloxy groups. Exemplary halogen atoms include fluorine, chlorine, and bromine atoms. Preferred substituents on the ring Z² include lower alkyl groups having about 1 to about 4 carbon atoms, carboxyl group, substituted oxycarbonyl groups, nitro group, and halogen atoms.

It is especially preferred in Formula (1) that (i) "n" is 0 and the ring Z² is a six-membered aromatic or nonaromatic carbon ring possessing one side in common with the cyclic imide skeleton Z¹, or that (ii) "n" is 1 and the ring Z² is naphthalene ring or decahydronaphthalene ring possessing two sides in common with the cyclic imide skeleton Z¹.

Of the immobilized cyclic imide catalysts represented by Formula (1), representative structures of the cyclic imide skeleton Z¹ moiety to which the ring Z² may be adjacent include the following structures, in which X is as defined above; and "A" in Formula (g) represents methylene group or oxygen atom .

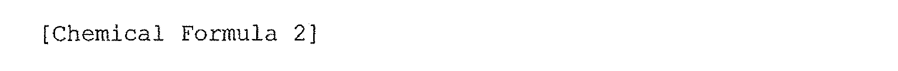

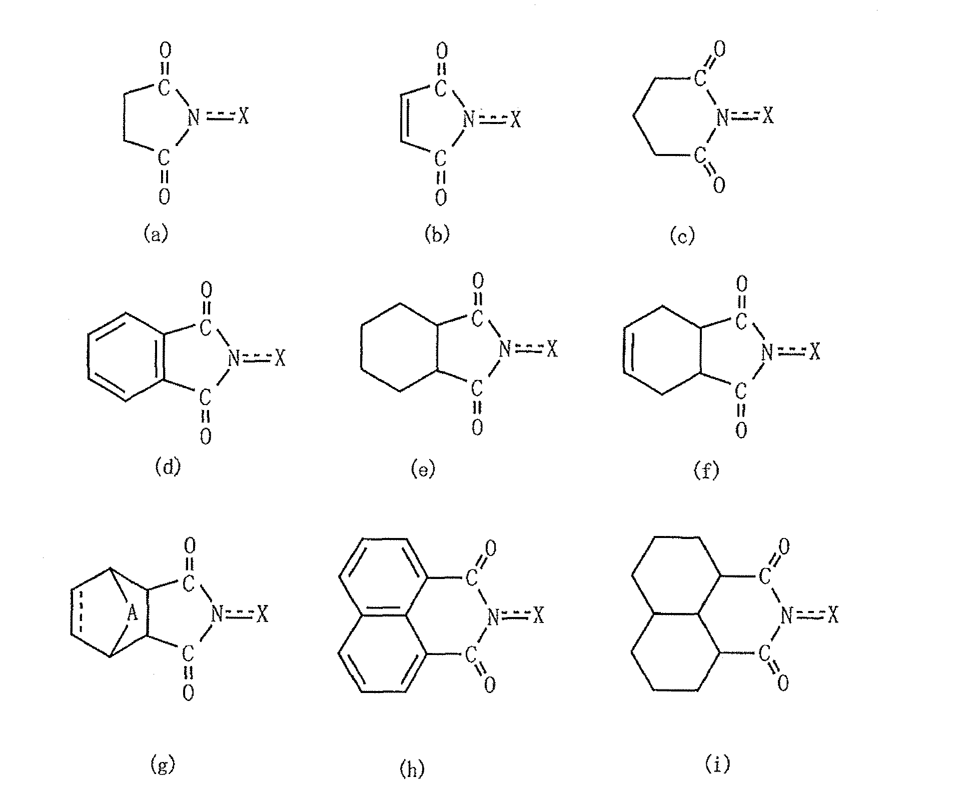

Representative examples of preferred skeletons as the cyclic imide skeleton Z¹ to which the ring Z² may be adjacent include skeletons in which the cyclic imide skeleton Z¹ is a five-membered ring, such as N-hydroxysuccinimide skeleton, N-hydroxymaleimide skeleton, N-hydroxyhexahydrophthalimide skeleton, N,N'-dihydroxycyclohexanetetracarboxylic diimide skeleton, N-hydroxyphthalimide skeleton, N-hydroxyhimimide skeleton, N,N'-dihydroxypyromellitic diimide skeleton, and N,N'-dihydroxynaphthalenetetracarboxylic diimide skeleton; and skeletons in which the cyclic imide skeleton Z¹ is a six-membered ring, such as N-hydroxyglutarimide skeleton, N-hydroxy-1,8-decahydronaphthalenedicarboximide skeleton, N,N'-dihydroxy-1,8;4,5-decahydronaphthalenetetracarboxylic diimide skeleton, N-hydroxy-1,8-naphthalenedicarboximide skeleton (N-hydroxynaphthalimide skeleton), and N,N'-dihydroxy-1,8;4,5-naphthalenetetracarboxylic diimide skeleton.

In Formula (1), elliptically shaped moiety "S" represents an inorganic support. The inorganic support S is preferably porous. Examples of the inorganic support S include a silica, an alumina, a titania, a zirconia, and a ceria. Each of different inorganic supports can be used alone or in combination. Exemplary inorganic supports further include multicomponent oxides of two or more elements selected from the group consisting of silicon, aluminum, zirconium, and cerium. Usable exemplary multicomponent oxides include silica-alumina, silica-titania, silica-zirconia, silica-ceria, and zeolite. Among them, preferred are a silica or a multicomponent oxide containing silicon element. The silica may be a silica whose particle diameter and pore size are precisely controlled, but it can also be a silica gel for use in column chromatography or a hygroscopic silica compound (hygroscopic silica gel).

The inorganic support S may be one whose surface has been activated. The surface of the inorganic support S generally has an active functional group (e.g., -OH group; or silanol group in the case of a silica). The active functional group of the surface of the inorganic support S may be bound to a terminal functional group of the linkage group A¹ through a siloxane bond or through another bonding.

Though not especially limited in shape, the inorganic support S is preferably in the form of a pellet or powder. The size thereof is, for example, from 10 nm to 10 mm, and preferably from 0.1 to 10 mm, in terms of diameter (or in terms of major axis). The pore size and distribution thereof of the inorganic support S are not especially limited.

In Formula (1), A¹ represents a linkage group linking the inorganic support S with the cyclic imide skeleton Z¹ or with the ring Z² and is either a bivalent hydrocarbon group or a group composed of a bivalent hydrocarbon group and an amide bond (-NHCO-). The carbon number of the linkage group A¹ is, for example, from about 1 to about 1000, preferably from about 1 to about 100, and more preferably from about 1 to about 20.

Exemplary bivalent hydrocarbon groups include bivalent aliphatic hydrocarbon groups including linear or branched-chain alkylene groups such as methylene, ethylene, propylene, isopropylidene, trimethylene, tetramethylene, pentamethylene, hexamethylene, octamethylene, decamethylene, dodecamethylene, tetradecamethylene, pentadecamethylene, hexadecamethylene, and octadecamethylene groups; bivalent alicyclic hydrocarbon groups such as 1,2-cyclopentylene, 1,3-cyclopentylene, cyclopentylidene, 1,2-cyclohexylene, 1,3-cyclohexylene, 1,4-cyclohexylene, and cyclohexylidene groups; bivalent aromatic hydrocarbon groups such as 1,2-phenylene, 1,3-phenylene, and 1,4-phenylene groups; and bivalent groups each composed of two or more of these groups bound to each other.

The bivalent hydrocarbons may each have substituents. Exemplary substituents include halogen atoms such as fluorine atom, chlorine atom, and bromine atom, of which fluorine atom is preferred; alkyl groups such as methyl, ethyl, propyl, isopropyl, butyl, isobutyl, s-butyl, t-butyl, and hexyl groups, of which alkyl groups having 1 to 12 carbon atoms are preferred, and alkyl groups having 1 to 6 carbon atoms are more preferred; cycloalkyl groups such as cyclopentyl and cyclohexyl groups, of which three- to fifty-membered cycloalkyl groups are preferred, and five- or six-membered cycloalkyl groups are more preferred; aryl groups such as phenyl and naphthyl groups; haloalkyl groups (of which fluoroalkyl groups are preferred), such as trifluoromethyl, pentafluoroethyl, and 2,2,2-trifluoroethyl groups, of which haloalkyl groups having 1 to 12 carbon atoms are preferred, and haloalkyl groups having 1 to 8 carbon atoms are more preferred; alkoxy groups such as methoxy, ethoxy, and propoxy groups, of which alkoxy groups having 1 to 12 carbon atoms are preferred, and alkoxy groups having 1 to 6 carbon atoms are more preferred; protected or unprotected hydroxyl group; protected or unprotected carboxyl groups [including substituted oxycarbonyl groups (e.g., alkoxy-carbonyl groups whose alkoxy moiety having 1 to 4 carbon atoms), such as methoxycarbonyl and ethoxycarbonyl groups]; cyano group; and silyl groups such as -SiY¹Y²Y³ groups mentioned below.

In the linkage group composed of a bivalent hydrocarbon group and an amide bond, the amide bond (-NHCO-) may face either side. Specifically, the carbonyl group of the amide bond may face either the cyclic imide skeleton Z¹ or the inorganic support S. The amide bond in the linkage group A¹ may lie at a terminus adjacent to the cyclic imide skeleton Z¹ or at an intermediate position, but it preferably lies at a terminus adjacent to the cyclic imide skeleton Z¹. In this case, the carbonyl group of the amide bond is preferably but not limitatively bound to an atom, such as carbon atom, constituting the cyclic imide skeleton Z¹ or bound to an atom, such as carbon atom, constituting the ring Z² adjacent to the cyclic imide skeleton Z¹. The linkage group may contain two or more of bivalent hydrocarbon groups and amide groups, respectively.

The linkage group A¹ may contain bivalent hydrocarbon group(s) (which may be substituted) alone, or contain bivalent hydrocarbon group(s) (which may be substituted) and amide bond(s) alone, but it may further contain one or more moieties such as carbonyl group, epoxy group, ether bond, thioether bond, ester bond, imide bond, urethane bond, urea bond, phosphoric ester bond, and siloxane bond, within ranges not adversely affecting the catalytic performance.

Though not especially limited, the amount of the cyclic imide skeleton Z¹ in the immobilized cyclic imide catalyst having a structure represented by Formula (1) is, for example, from 0.001 mmol to 20 mmol, preferably from 0.01 mmol to 2 mmol, and more preferably from 0.05 mmol to 0.5 mmol, per 1 g of the inorganic support.

Each of different immobilized cyclic imide catalysts having a structure represented by Formula (1) can be used alone or in combination. Such immobilized cyclic imide catalysts having a structure represented by Formula (1) may be formed within the reaction system.

The amount of immobilized cyclic imide catalysts having a structure represented by Formula (1) can be chosen within a broad range, and is, in terms of the amount of the cyclic imide skeleton Z¹, for example, from about 0.0000001 to about 1 mole, preferably from about 0.00001 to about 0.5 mole, more preferably from about 0.0001 to about 0.4 mole, and especially preferably from about 0.001 to about 0.35 mole, per 1 mole of the reaction component (substrate).

### [Preparation of Immobilized Cyclic Imide Catalysts]

Of immobilized cyclic imide catalysts having a structure represented by Formula (1), immobilized catalysts in which the linkage group A¹ is a group composed of a bivalent hydrocarbon group and an amide bond can be prepared, for example, by reacting an inorganic support S represented by Formula (2), a silane coupling agent represented by Formula (3), and a carboxylic acid containing a cyclic imide skeleton and represented by Formula (4), or a reactive derivative thereof (e.g., an acyl halide, an acid anhydride, or an ester), as in the following scheme:

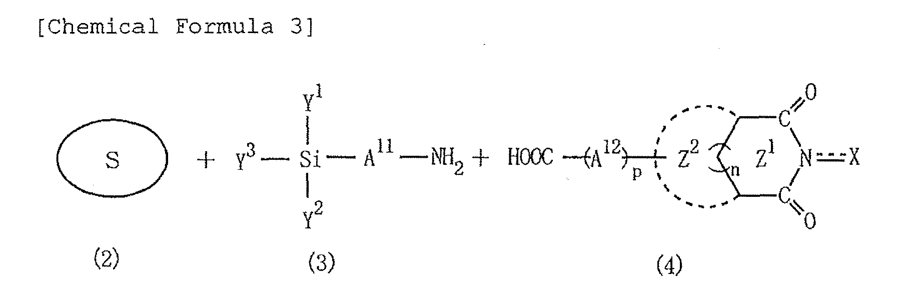

In the scheme, Y¹ and Y² each represent hydroxyl group, an alkoxy group, a halogen atom, or an alkyl group; Y³ represents hydroxyl group, an alkoxy group, or a halogen atom; A¹¹ and A¹² each represent a bivalent hydrocarbon group; "p" denotes 0 or 1; and X, Z¹, Z², and elliptically shaped moiety "S" are as defied above. The compound of Formula (4) may have, per molecule, two or more of the cyclic imide skeleton Z¹ to which the ring Z² may be adjacent.

Exemplary alkoxy groups as Y¹ to Y³ include alkoxy groups having about 1 to about 6 carbon atoms, such as methoxy, ethoxy, propoxy, isopropyloxy, butoxy, isobutyloxy, t-butyloxy, pentyloxy, and hexyloxy groups, of which alkoxy groups having 1 to 4 carbon atoms are preferred. Exemplary halogen atoms include chlorine atom and bromine atom. Exemplary alkyl groups as Y¹ and Y² include linear or branched-chain alkyl groups having about 1 to about 18 carbon atoms, such as methyl, ethyl, propyl, isopropyl, butyl, isobutyl, s-butyl, t-butyl, pentyl, hexyl, octyl, decyl, and dodecyl groups, of which alkyl groups having 1 to 10 carbon atoms are preferred, and alkyl groups having 1 to 6 carbon atoms are more preferred. Exemplary bivalent hydrocarbon groups as A¹¹ and A¹² are as with those mentioned in the description of A¹.

The compound represented by Formula (3) may have two or more -SiY¹Y²Y³ groups and two or more amino groups, respectively, per molecule. In this case, the two or more - SiY¹Y²Y³ groups may be the same as or different from one another. The compound represented by Formula (4) may contain two or more carboxyl groups or equivalent functional groups thereto (e.g., acyl halide groups, acid anhydride groups, and alkoxycarbonyl groups). When the compound has two or more carboxyl groups or equivalent functional groups thereof, they may be the same as or different from one another.

In the above process, reactions may be performed in any order not especially limited. It is acceptable that the inorganic support S represented by Formula (2) is reacted with the silane coupling agent represented by Formula (3) (a silane coupling reaction is performed), and thereafter the carboxylic acid containing a cyclic imide skeleton and represented by Formula (4) or a reactive derivative thereof is reacted to form an amide bond; that the silane coupling agent represented by Formula (3) is reacted with the carboxylic acid containing a cyclic imide skeleton and represented by Formula (4) or a reactive derivative thereof to form an amide bond, and thereafter the inorganic support S represented by Formula (2) is reacted therewith (a silane coupling reaction is performed); or that the three components are reacted simultaneously.

The silane coupling reaction can be carried out according to a known procedure for reacting a silane coupling agent with an inorganic substance. A combination use typically of diethoxy(dimethyl)silane with the silane coupling agent upon the silane coupling reaction enables precise control of the amount to be supported by the inorganic support (the amount of bonding silane coupling agent). The reaction for the formation of an amide bond can be performed according to a common or regular procedure for reacting an amine with a carboxylic acid or a reactive derivative thereof.

It is also acceptable that a reaction is performed using a carboxylic acid containing a precursor skeleton to a cyclic imide skeleton, or a reactive derivative thereof, instead of, or in combination as a mixture with, the carboxylic acid containing a cyclic imide skeleton and represented by Formula (4) or a reactive derivative thereof, and the precursor skeleton to the cyclic imide skeleton is converted into the cyclic imide skeleton in a suitable stage.

Exemplary precursor skeletons to cyclic imide skeletons include the following skeletons: 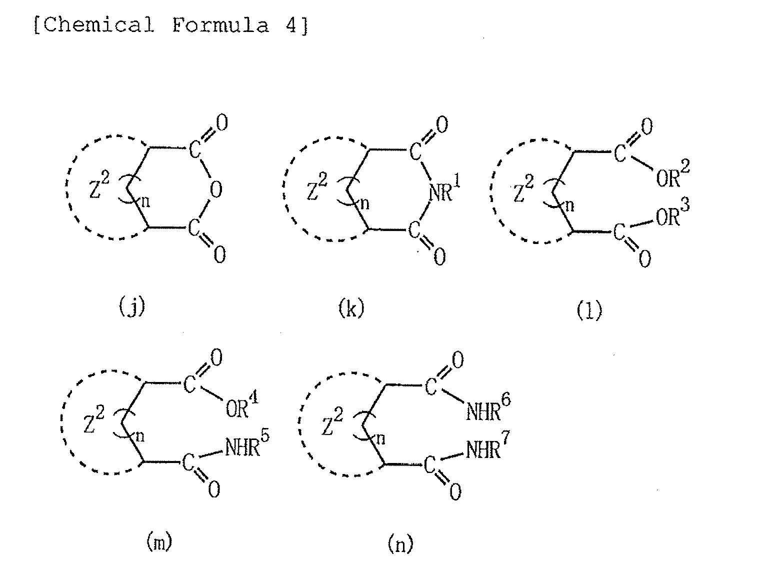 wherein R¹, R², R³, R⁴, R⁵, R⁶, and R⁷ are the same as or different from one another and each represent hydrogen atom or a hydrocarbon group; the ring Z² and "n" are as defined above; and the ring Z² may either be present or not.

Examples of the hydrocarbon group include alkyl groups (e.g., alkyl groups having 1 to 6 carbon atoms), such as methyl, ethyl, propyl, isopropyl, butyl, isobutyl, s-butyl, and t-butyl groups; cycloalkyl groups such as cyclopentyl group; aryl groups such as phenyl group; and aralkyl groups such as benzyl group.

Each of R¹, R², R³, R⁴, R⁵, R⁶, and R⁷ are not limited to the above-listed groups, as long as being a group that can be converted into a compound having a cyclic imide skeleton (cyclic imide compound) by reacting a compound having a precursor skeleton to a cyclic imide skeleton (cyclic imide compound precursor) with a hydroxylamine (H₂N-OR; wherein R is as defined above) whose oxygen atom may be protected, or a slat thereof (an organic salt or inorganic salt).

Or immobilized cyclic imide catalysts having a structure represented by Formula (1), solid catalysts (immobilized catalysts) in which the linkage group A¹ is a bivalent hydrocarbon group can be prepared, for example, by reacting an inorganic support S represented by Formula (2) and a compound represented by Formula (5) in which a reactive silyl group (e.g., a hydrolyzable silyl group) is bound to the cyclic imide skeleton Z¹ or the ring Z² adjacent thereto, through a hydrocarbon group, as illustrated in the following scheme:

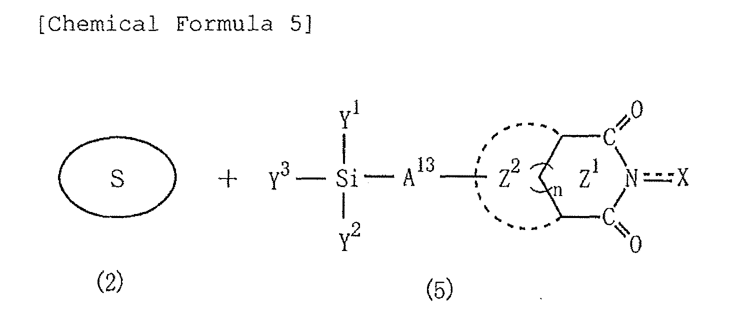

In the scheme, A¹³ represents a bivalent hydrocarbon group; and X, Z¹, Z², Y¹, Y², Y³, and elliptically shaped moiety "S" are as defined above. The compound of Formula (5) may contain, per molecule, two or more of the cyclic imide skeleton Z¹ to which the ring Z² may be adjacent. Exemplary bivalent hydrocarbon groups as A¹³ are as with those exemplified in the description of A¹. The reaction can be performed according to a known procedure for reacting a silane coupling agent with an inorganic substance.

As is described above, it is also acceptable that a reaction is performed using a corresponding compound containing a precursor skeleton to a cyclic imide skeleton instead of, or in combination as a mixture with, the compound represented by Formula (5), and the precursor skeleton to the cyclic imide skeleton is converted into the cyclic imide skeleton in a suitable stage.

The way to bond or link the inorganic support S with the linkage group A¹ is not limited to a silane coupling reaction and is not especially limited, as long as being a reaction that can form a covalent bond. The inorganic support S and the linkage group A¹ can be bound to each other by using a reaction component that contains the linkage group A¹ and has a functional group at the terminal of the linkage group A¹, which functional group is capable of reacting with a functional group of the surface of the inorganic support S.

Of immobilized cyclic imide catalysts having a structure represented by Formula (1), a catalyst in which X is an -OR group and R is hydrogen atom, and another catalyst in which X is an -OR group and R is a hydroxyl-protecting group can be converted into each other through a common reaction for introducing a protecting group or through a common deprotection reaction.

### [Promoters]

A promoter (co-catalyst) may be used herein in combination with the immobilized cyclic imide catalyst(s) having a structure represented by Formula (1). Exemplary promoters include metallic compounds. The combination use of the catalyst(s) with metallic compound(s) enables improvements in rate and selectivity of the reaction.

Metallic elements constituting such metallic compounds are not especially limited, but they are often metallic elements belonging to Groups 1 to 15 of the Periodic Table. As used herein the term "metallic elements" also means and includes boron B. Examples of the metallic elements include, of the Periodic Table, Group 1 elements (e.g., Li, Na, and K), Group 2 elements (e.g., Mg, Ca, Sr, and Ba), Group 3 elements (e.g., Sc, lanthanoid elements, and actinoid elements), Group 4 elements (e.g., Ti, Zr, and Hf), Group 5 elements (e.g., V), Group 6 elements (e.g., Cr, Mo, and W), Group 7 elements (e.g., Mn), Group 8 elements (e.g., Fe and Ru), Group 9 elements (e.g., Co and Rh), Group 10 elements (e.g., Ni, Pd, and Pt), Group 11 elements (e.g., Cu), Group 12 elements (e.g., Zn), Group 13 elements (e.g., B, Al, and In), Group 14 elements (e.g., Sn and Pb), and Group 15 elements (e.g., Sb and Bi). Preferred metallic elements include transition metal elements (elements belonging to Groups 3 to 12 of the Periodic Table) and elements belonging to Group 13 of the Periodic Table (e.g., In). Among them, elements belonging to Groups 5 to 11 of the Periodic Table are preferred, of which elements belonging to Groups 5 to 9 are more preferred, and V, Mo, Mn, and Co are especially typically preferred. The metallic elements may have any valence not especially limited. They may have a valence of, for example, from about 0 to about 6.

Exemplary metallic compounds include, of the metallic elements, inorganic compounds including elementary substances, hydroxides, oxides (including multicomponent oxides), halides (fluorides, chlorides, bromides, and iodides), salts of oxoacids (e.g., nitrates, sulfates, phosphates, borates, and carbonates), salts of isopolyacids, and salts of heteropolyacids; and organic compounds including salts of organic acids (e.g., acetates, propionates, prussiates (cyanides), naphthenates, and stearates), and complexes. Exemplary ligands for constituting the complexes include OH (hydroxo), alkoxys (e.g., methoxy, ethoxy, propoxy, and butoxy), acyls (e.g., acetyl and propionyl), alkoxycarbonyls (e.g., methoxycarbonyl and ethoxycarbonyl), acetylacetonato, cyclopentadienyl group, halogen atoms (e.g., chlorine and bromine), CO, CN, oxygen atom, H₂O (aquo); phosphines (e.g., triarylphosphines such as triphenylphosphine) and other phosphorus compounds; and NH₃ (ammine), NO, NO₂ (nitro), NO₃ (nitrato), ethylenediamine, diethylenetriamine, pyridine, phenanthroline, and other nitrogen-containing compounds.

Taking cobalt compounds as an example, specific examples of the metallic compounds include bivalent or trivalent cobalt compounds including inorganic compounds such as cobalt hydroxide, cobalt oxide, cobalt chloride, cobalt bromide, cobalt nitrate, cobalt sulfate, and cobalt phosphate; salts of organic acids, such as cobalt acetate, cobalt naphthenate, and cobalt stearate; and complexes such as cobalt acetylacetonate. Exemplary vanadium compounds include vanadium compounds having a valence of 2 to 5 (bivalent to pentavalent vanadium compounds), including inorganic compounds such as vanadium hydroxide, vanadium oxide, vanadium chloride, vanadyl chloride, vanadium sulfate, vanadyl sulfate, and sodium vanadate; and complexes such as vanadium acetylacetonate and vanadyl acetylacetonate. Exemplary compounds of other metallic elements include compounds corresponding to the cobalt or vanadium compounds. Each of different metallic compounds can be used alone or in combination. Above all, a combination use of a cobalt compound and a manganese compound may often significantly improve the reaction rate. A combination use of two or more metallic compounds having different valances (e.g., a bivalent metallic compound and a trivalent metallic compound) is also preferred.

The amount of the metallic compounds is, for example, from about 0.001 to about 10 moles, and preferably from about 0.005 to about 3 moles, per 1 mole of the cyclic imide skeleton Z¹ in the immobilized cyclic imide catalyst having a structure represented by Formula (1). The amount of the metallic compounds is, for example, from about 0.00001 percent by mole to about 10 percent by mole, and preferably from about 0.2 percent by mole to about 2 percent by mole, per 1 mole of the reaction component (substrate).

Exemplary promoters usable in the present invention further include organic salts each containing a polyatomic cation or a polyatomic anion and its counter ion, which polyatomic cation or anion contains a Group 15 or Group 16 element of the Periodic Table having at least one organic group bound therewith. Use of the organic salts as promoters can further improve the rate and selectivity of the reaction.

In the organic salts, examples of Group 15 elements of the Periodic Table include N, P, As, Sb, and Bi. Examples of Group 16 elements of the Periodic Table include O, S, Se, and Te. Preferred elements include N, P, As, Sb, and S, of which N, P, and S are typically preferred. Exemplary organic groups to be bound to atoms of the elements include hydrocarbon groups which may be substituted, and substituted oxy groups. Exemplary hydrocarbon groups include linear or branched-chain aliphatic hydrocarbon groups (alkyl groups, alkenyl groups, and alkynyl groups) having about 1 to about 30 carbon atoms, of which those having about 1 to about 20 carbon atoms are preferred; alicyclic hydrocarbon groups having about 3 to about 8 carbon atoms; and aromatic hydrocarbon groups having about 6 to about 14 carbon atoms. Examples of the substituted oxy groups include alkoxy groups, aryloxy groups, and aralkyloxy groups.

Representative examples of the organic salts include organic onium salts such as organic ammonium salts, organic phosphonium salts, and organic sulfonium salts. Specific examples of organic ammonium salts include quaternary ammonium salts whose nitrogen atom has four hydrocarbon groups bound thereto, including quaternary ammonium chlorides such as tetramethylammonium chloride, tetraethylammonium chloride, tetrabutylammonium chloride, tetrahexylammonium chloride, trioctylmethylammonium chloride, triethylphenylammonium chloride, tributyl (hexadecyl)ammonium chloride, and di(octadecyl)dimethylammonium chloride, and corresponding quaternary ammonium bromides; and cyclic quaternary ammonium salts such as dimethylpiperidinium chloride, hexadecylpyridinium chloride, and methylquinolinium chloride. Specific examples of organic phosphonium salts include quaternary phosphonium salts whose phosphorus atom has four hydrocarbon groups bound thereto, including quaternary phosphonium chlorides such as tetramethylphosphonium chloride, tetrabutylphosphonium chloride, tributyl (hexadecyl)phosphonium chloride, and triethylphenylphosphonium chloride, and corresponding quaternary phosphonium bromides. Specific examples of organic sulfonium salts include sulfonium salts whose sulfur atom has three hydrocarbon groups bound thereto, such as triethylsulfonium iodide and ethyldiphenylsulfonium iodide.

Examples of the organic salts further include alkyl-substituted sulfonates (e.g., alkyl-substituted sulfonates whose alkyl moiety having 6 to 18 carbon atoms), such as methanesulfonates, ethanesulfonates, octanesulfonates, and dodecanesulfonates; aryl-substituted sulfonates whose aryl moiety may be substituted with alkyl group(s) (e.g., alkylarylsulfonates whose alkyl moiety having 6 to 18 carbon atoms), such as benzenesulfonates, p-toluenesulfonates, naphthalenesulfonates, decylbenzenesulfonates, and dodecylbenzenesulfonates; sulfonic acid type ion exchange resins (ion exchangers); and phosphonic acid type ion exchange resins (ion exchangers).

The amount of organic salts is, for example, from about 0.001 to about 0.1 mole, and preferably from about 0.005 to about 0.08 mole, per 1 mole of the cyclic imide skeleton Z¹ in the immobilized cyclic imide catalyst having a structure represented by Formula (1).

Exemplary promoters usable in the present invention further include strong acids (e.g., compounds having a pKa of 2 or less at 25°C). Preferred exemplary strong acids include hydrogen halides, hydrohalic acids, sulfuric acid, and heteropolyacids. The amount of strong acids is, for example, from about 0.001 to about 3 moles, per 1 mole of the cyclic imide skeleton Z¹ in the immobilized cyclic imide catalyst having a structure represented by Formula (1).

Exemplary promoters usable in the present invention further include compounds having a carbonyl group to which an electron-withdrawing group is bound. Representative examples of such compounds having a carbonyl group to which an electron-withdrawing group is bound include hexafluoroacetone, trifluoroacetic acid, pentafluorophenyl methyl ketone, pentafluorophenyl trifluoromethyl ketone, and benzoic acid. The amount of these compounds is, for example, from about 0.0001 to about 3 moles, per 1 mole of the reaction component (substrate).

The reaction system herein may contain a free-radical generator (e.g., a free-radical initiator) and/or a free-radical reaction accelerator. Examples of such components include halogens such as chlorine and bromine; peracids such as peracetic acid and m-chloroperbenzoic acid; peroxides including hydroperoxides, such as hydrogen peroxide and t-butyl hydroperoxide (TBHP); azo compounds such as azobisisobutyronitrile; acetophenones; cyclic amine-N-oxyl compounds; nitric acid or nitrous acid, or salts of them; nitrogen dioxide; and aldehydes such as benzaldehyde. The presence of the component in the system may accelerate the reaction. The amount of the component is, for example, from about 0.0001 to about 0.7 mole, and preferably from about 0.001 to about 1 mole, per 1 mole of the cyclic imide skeleton Z¹ in the immobilized cyclic imide catalyst having a structure represented by Formula (1).

Catalysts according to the present invention are useful typically as catalysts for free-radical reactions. They are advantageously usable in heterogeneous reactions, because they have catalytic activities as with a known catalyst N-hydroxyphthalimide and, additionally, are solid catalysts. Accordingly, they can be easily separated and recovered from a reaction mixture and can be easily separated from a reaction product after the completion of reaction. The recovered catalysts, if being deactivated due typically to deterioration or decomposition, can be regenerated according to a simple procedure, and the regenerated catalysts can be recycled and reused in the reaction system.

### [Process for Oxidation of Organic Compounds]

A process for the oxidation of organic compounds, according to the present invention, includes carrying out oxidation of an organic compound in the presence of the immobilized cyclic imide catalyst having a structure represented by Formula (1), and where necessary, the promoter, to give an oxidation reaction product (oxidized product). As an oxidizing agent, oxygen is generally used.

The organic compound used as a reaction material (substrate) is not especially limited, as long as being a compound that is oxidizable with oxygen in the presence of such an imide compound catalyst. Compounds (A) capable of forming stable free radicals are preferred as substrates. Representative examples of such compounds include (A1) heteroatom-containing compounds each having a carbon-hydrogen bond at an adjacent position to the heteroatom, (A2) compounds each having a carbon-heteroatom double bond, (A3) compounds each having methine carbon atom, (A4) compounds each having a carbon-hydrogen bond at an adjacent position to an unsaturated bond, (A5) nonaromatic cyclic hydrocarbons, (A6) conjugated compounds, (A7) amines, (A8) aromatic compounds, (A9) linear alkanes, and (A10) olefins.

Each of these compounds may have one or more substituents within ranges not inhibiting the reaction. Exemplary substituents include halogen atoms, hydroxyl group, mercapto group, oxo group, substituted oxy groups (e.g., alkoxy groups, aryloxy groups, and acyloxy groups), substituted thio groups, carboxyl group, substituted oxycarbonyl groups, substituted or unsubstituted carbamoyl groups, cyano group, nitro group, substituted or unsubstituted amino groups, sulfo group, alkyl groups, alkenyl groups, alkenyl groups, alicyclic hydrocarbon groups, aromatic hydrocarbon groups, and heterocyclic groups.

Exemplary heteroatom-containing compounds (A1) each having a carbon-hydrogen bond at an adjacent position to the heteroatom include (A1-1) primary or secondary alcohols and primary or secondary thiols; (A1-2) ethers each having a carbon-hydrogen bond at an adjacent position to the oxygen atom, and sulfides each having a carbon-hydrogen bond at an adjacent position to the sulfur atom; and (A1-3) acetals (including hemiacetals) each having a carbon-hydrogen bond at an adjacent position to the oxygen atom, and thioacetals (including thiohemiacetals) each having a carbon-hydrogen bond at an adjacent position to the sulfur atom.

The primary or secondary alcohols as the compounds (A1-1) include a wide variety of alcohols. These alcohols may be whichever of monohydric, dihydric, and polyhydric alcohols.

Representative examples of primary alcohols include saturated or unsaturated aliphatic primary alcohols having about 1 to about 30 carbon atoms, such as methanol, ethanol, 1-propanol, 1-butanol, 1-pentanol, 1-hexanol, 1-octanol, 1-decanol, 2-buten-1-ol, ethylene glycol, trimethylene glycol, hexamethylene glycol, and pentaerythritol, of which those having about 1 to about 20 carbon atoms are preferred, and those having about 1 to about 15 carbon atoms are more preferred; saturated or unsaturated alicyclic primary alcohols such as cyclopentylmethyl alcohol, cyclohexylmethyl alcohol, and 2-cyclohexylethyl alcohol; aromatic primary alcohols such as benzyl alcohol, 2-phenylethyl alcohol, 3-phenylpropyl alcohol, and cinnamic alcohol; and heterocyclic alcohols such as 2-hydroxymethylpyridine.

Representative examples of secondary alcohols include saturated or unsaturated aliphatic secondary alcohols having about 3 to about 30 carbon atoms, such as 2-propanol, s-butyl alcohol, 2-pentanol, 2-octanol, 2-pentene-4-ol, as well as vicinal diols such as 1,2-propanediol, 2,3-butanediol, and 2,3-pentanediol, of which those having about 3 to about 20 carbon atoms are preferred, and those having about 3 to about 15 carbon atoms are more preferred; secondary alcohols each having an aliphatic hydrocarbon group and an alicyclic hydrocarbon group (e.g., a cycloalkyl group) bound to a hydroxyl-binding carbon atom, such as 1-cyclopentylethanol and 1-cyclohexylethanol; saturated or unsaturated alicyclic secondary alcohols (including bridged cyclic secondary alcohols) having about 3 to about 20 members, such as cyclopentanol, cyclohexanol, cyclooctanol, cyclododecanol, 2-cyclohexen-1-ol, 2-adamantanol, 2-adamantanols having one to four hydroxyl groups at the bridgehead positions, and 2-adamantanols having oxo group on the adamantane ring, of which those having about 3 to about 15 members are preferred, those having about 5 to about 15 members are more preferred, and those having about 5 to about 8 members are especially preferred; aromatic secondary alcohols such as 1-phenylethanol; heterocyclic secondary alcohols such as 1-(2-pyridyl)ethanol.

Representative examples of alcohols further include alcohols having a bridged cyclic hydrocarbon group (e.g., compounds having a bridged cyclic hydrocarbon group bound to a hydroxyl-binding carbon atom), such as 1-adamantanemethanol, α-methyl-1-adamantanemethanol, 3-hydroxy-α-methyl-1-adamantanemethanol, 3-carboxy-α-methyl-1-adamantanemethanol, α-methyl-3a-perhydroindenemethanol, α-methyl-4a-decahydronaphthalenemethanol, α-methyl-4a-perhydrofluorenemethanol, α-methyl-2-tricyclo[5.2.1.0^{2,6}]decanemethanol, and α-methyl-1-norbornanemethanol.

Preferred alcohols include secondary alcohols and the alcohols having a bridged cyclic hydrocarbon group. Exemplary preferred secondary alcohols include aliphatic secondary alcohols such as 2-propanol and s-butyl alcohol; secondary alcohols each having an aliphatic hydrocarbon group (e.g., an alkyl group having 1 to 4 carbon atoms or an aryl group having 6 to 14 carbon atoms) and a nonaromatic carbocyclic group (e.g., a cycloalkyl or cycloalkenyl group having 3 to 15 carbon atoms) bound to a hydroxyl-binding carbon atom, such as 1-cyclohexylethanol; alicyclic secondary alcohols having about 3 to about 15 members, such as cyclopentanol, cyclohexanol, and 2-adamantanol; and aromatic secondary alcohols such as 1-phenylethanol.

Exemplary primary or secondary thiols as the compounds (A1-1) include thiols corresponding to the primary or secondary alcohols.

Exemplary ethers each having a carbon-hydrogen bond at an adjacent position to the oxygen atom as the compounds (A1-2) include aliphatic ethers such as dimethyl ether, diethyl ether, dipropyl ether, diisopropyl ether, dibutyl ether, and diallyl ether; aromatic ethers such as anisole, phenetole, dibenzyl ether, and phenyl benzyl ether; and cyclic ethers (to which an aromatic ring or nonaromatic ring may be fused), such as dihydrofuran, tetrahydrofuran, pyran, dihydropyran, tetrahydropyran, morpholine, chroman, and isochroman.

Exemplary sulfides each having a carbon-hydrogen bond at an adjacent position to the sulfur atom as the compounds (A1-2) include sulfides corresponding to the exemplified ethers having a carbon-hydrogen bond at an adjacent position to the oxygen atom.

Exemplary acetals each having a carbon-hydrogen bond at an adjacent position to the oxygen atom as the compounds (A1-3) include acetals derived from aldehydes and alcohols or acid anhydrides. Such acetals include cyclic acetals and acyclic acetals. The aldehydes include aliphatic aldehydes such as formaldehyde, acetaldehyde, propionaldehyde, butylaldehyde, and isobutylaldehyde; alicyclic aldehydes such as cyclopentanecarbaldehyde and cyclohexanecarbaldehyde; and aromatic aldehydes such as benzaldehyde and phenylacetaldehyde. Examples of the alcohol include monohydric alcohols such as methanol, ethanol, 1-propanol, 1-butanol, and benzyl alcohol; and dihydric alcohols such as ethylene glycol, propylene glycol, 1,3-propanediol, and 2,2-dibromo-1,3-propanediol. Exemplary representative acetals include 1,3-dioxolane compounds such as 1,3-dioxolane, 2-methyl-1,3-dioxolane, and 2-ethyl-1,3-dioxolane; 1,3-dioxane compounds such as 2-methyl-1,3-dioxane; and dialkylacetal compounds such as acetaldehyde dimethyl acetal.

Exemplary thioacetals having a carbon-hydrogen bond at an adjacent position to the sulfur atom as the compounds (A1-3) include thioacetals corresponding to the above-mentioned acetals having a carbon-hydrogen bond at an adjacent position to the oxygen atom.

Examples of the compounds (A2) each having a carbon-heteroatom double bond include (A2-1) carbonyl-containing compounds; (A2-2) thiocarbonyl-containing compounds; and (A2-3) imines. The carbonyl-containing compounds (A2-1) include ketones and aldehydes. Exemplary ketones and aldehydes include chain ketones such as acetone, methyl ethyl ketone, methyl isobutyl ketone, 3-pentanone, methyl vinyl ketone, methyl cyclohexyl ketone, and acetophenone; cyclic ketones such as cyclopentanone, cyclohexanone, 4-methylcyclohexanone, isophorone, cyclodecanone, cyclododecanone, 1,4-cyclooctanedione, 2,2-bis(4-oxocyclohexyl)propane, and 2-adamantanone; 1,2-dicarbonyl compounds (e.g., α-diketones), such as biacetyl (2,3-butanedione), bibenzoyl (benzil), acetylbenzoyl, and cyclohexane-1,2-dione; α-keto-alcohols such as acetoin and benzoin; aliphatic aldehydes such as acetaldehyde, propionaldehyde, butanal, hexanal, succinaldehyde, glutaraldehyde, and adipaldehyde; alicyclic aldehydes such as cyclohexyl aldehyde, citral, and citronellal; aromatic aldehydes such as benzaldehyde, carboxybenzaldehyde, nitrobenzaldehyde, cinnamaldehyde, salicylaldehyde, anisaldehyde, phthalaldehyde, isophthalaldehyde, and terephthalaldehyde; and heterocyclic aldehydes such as furfural and nicotinaldehyde.

Exemplary thiocarbonyl-containing compounds (A2-2) include thiocarbonyl-containing compounds corresponding to the above-mentioned carbonyl-containing compounds (A2-1).

Exemplary imines (A2-3) include imines (including oximes and hydrazones) derived from the carbonyl-containing compound (A2-1) and ammonia or amines. Exemplary amines include amines such as methylamine, ethylamine, propylamine, butylamine, hexylamine, benzylamine, cyclohexylamine, and aniline; hydroxylamines such as hydroxylamine and O-methylhydroxylamine; and hydrazines such as hydrazine, methylhydrazine, and phenylhydrazine.

The compounds (A3) each having methine carbon atom include (A3-1) cyclic compounds each having methine group (i.e., a methine carbon-hydrogen bond) as a constitutional unit of its ring; and (A3-2) chain compounds each having methine carbon atom.

Exemplary cyclic compounds (A3-1) include (A3-1a) bridged cyclic compounds each having at least one methine group; and (A3-1b) nonaromatic cyclic compounds (e.g., alicyclic hydrocarbons) each having a hydrocarbon group bound to its ring. The bridged cyclic compounds further include compounds each containing two rings having two carbon atoms in common, such as hydrogenated products of fused polycyclic aromatic hydrocarbons.

Exemplary bridged cyclic compounds (A3-1a) include bridged cyclic hydrocarbons or bridged heterocyclic compounds each having two to four rings, such as decahydronaphthalene, bicyclo[2.2.0]hexane, bicyclo[2.2.2]octane, bicyclo[3.2.1]octane, bicyclo[4.3.2]undecane, bicyclo[3.3.3]undecane, thujone, carane, pinane, pinene, bornane, bornylene, norbornane, norbornene, camphor, camphoric acid, camphene, tricyclene, tricyclo[5.2.1.0^{3,8}]decane, tricyclo[4.2.1.1^{2,5}]decane, exo-tricyclo[5.2.1.0^{2,6}]decane, endo-tricyclo[5.2.1.0^{2,6}]decane, tricyclo[4.3.1.1^{2,5}]undecane, tricyclo[4.2.2.1^{2,5}]undecane, endo-tricyclo[5.2.2.0^{2,6}]undecane, adamantane, 1-adamantanol, 1-chloroadamantane, 1-methyladamantane, 1,3-dimethyladamantane, 1-methoxyadamantane, 1-carboxyadamantane, 1-methoxycarbonyladamantane, 1-nitroadamantane, tetracyclo[4.4.0.1^{2,5}.1^{7,10}]dodecane, perhydroanthracene, perhydroacenaphthene, perhydrophenanthrene, perhydrophenalene, perhydroindene, and quinuclidine; and derivatives of them. These bridged cyclic compounds each have a methine carbon atom at a bridgehead position (corresponding to a junction site when two rings have two atoms in common).

Exemplary nonaromatic cyclic compounds (A3-1b) each having a hydrocarbon group bound to its ring include alicyclic hydrocarbons each having about 3 to about 15 members and containing a hydrocarbon group (e.g., an alkyl group) bound to its ring, and derivatives thereof, such as 1-methylcyclopentane, 1-methylcyclohexane, limonene, menthene, menthol, carvomenthone, and menthone. The hydrocarbon group just mentioned above may have about 1 to about 20 carbon atoms, and preferably about 1 to about 10 carbon atoms. The nonaromatic cyclic compounds (A3-1b) each having a hydrocarbon group bound to its ring have a methine carbon atom at the bonding site between the ring and the hydrocarbon group.

Exemplary chain compounds (A3-2) each having methine carbon atom include chain hydrocarbons each having a tertiary carbon atom, including aliphatic hydrocarbons having about 4 to about 20 (preferably about 4 to about 10) carbon atoms, such as isobutane, isopentane, isohexane, 3-methylpentane, 2,3-dimethylbutane, 2-methylhexane, 3-methylhexane, 3,4-dimethylhexane, and 3-methyloctane; and derivatives of them.

Examples of the compounds (A4) each having a carbon-hydrogen bond at an adjacent position to an unsaturated bond include (A4-1) aromatic compounds each having methyl group or methylene group at an adjacent position to an aromatic ring (at a "benzylic position"); and (A4-2) nonaromatic compounds each having methyl group or methylene group at an adjacent position to an unsaturated bond (e.g., carbon-carbon unsaturated bond or carbon-oxygen double bond).

The aromatic rings in the aromatic compounds (A4-1) may each be whichever of an aromatic hydrocarbon ring and an aromatic heterocyclic ring (heteroaromatic rings). Exemplary aromatic hydrocarbon rings include benzene ring and fused carbon rings. Exemplary fused carbon rings include fused carbon rings each having fused two to ten 4-to seven-membered carbon rings, such as naphthalene, azulene, indacene, anthracene, phenanthrene, triphenylene, and pyrene. Exemplary aromatic heterocyclic rings include heterocyclic rings containing oxygen atom as a heteroatom, including five-membered rings such as furan, oxazole, and isoxazole, six-membered rings such as 4-oxo-4H-pyran, and fused rings such as benzofuran, isobenzofuran, and 4-oxo-4H-chromene; heterocyclic rings containing sulfur atom as a heteroatom, including five-membered rings such as thiophene, thiazole, isothiazole, and thiadiazole, six-membered rings such as 4-oxo-4H-thiopyran, and fused rings such as benzothiophene; and heterocyclic rings containing nitrogen atom as a heteroatom, including five-membered rings such as pyrrole, pyrazole, imidazole, and triazole, six-membered rings such as pyridine, pyridazine, pyrimidine, and pyrazine, and fused rings such as indole, quinoline, acridine, naphthyridine, quinazoline, and purine.

The methylene group at an adjacent position to an aromatic ring may be a methylene group constituting a nonaromatic ring fused to the aromatic ring. The compounds (A4-1) may each have both methyl group and methylene group at an adjacent position to an aromatic ring.

Exemplary aromatic compounds each having methyl group at an adjacent position to an aromatic ring include aromatic hydrocarbons whose aromatic ring having about one to about six methyl groups substituted thereon, such as toluene, xylenes, 1-ethyl-4-methylbenzene, 1-ethyl-3-methylbenzene, 1-isopropyl-4-methylbenzene, 1-t-butyl-4-methylbenzene, 1-methoxy-4-methylbenzene, mesitylene, pseudocumene, durene, methylnaphthalene, dimethylnaphthalene, methylanthracene, 4,4'-dimethylbiphenyl, tolualdehyde, dimethylbenzaldehyde, trimethylbenzaldehyde, toluic acid, trimethylbenzoic acid, and dimethylbenzoic acid; and heterocyclic compounds whose heterocyclic ring having about one to about six methyl groups substituted thereon, such as 2-methylfuran, 3-methylfuran, 3-methylthiophene, 2-methylpyridine, 3-methylpyridine, 4-methylpyridine, 2,4-dimethylpyridine, 2,4,6-trimethylpyridine, 4-methylindole, 2-methylquinoline, and 3-methylquinoline.

Exemplary aromatic compounds having methylene group at an adjacent position to an aromatic ring include aromatic hydrocarbons each containing an alkyl group or substituted alkyl group having 2 or more carbon atoms, such as ethylbenzene, propylbenzene, 1,4-diethylbenzene, and diphenylmethane; aromatic heterocyclic compounds each containing an alkyl group or substituted alkyl group having 2 or more carbon atoms, such as 2-ethylfuran, 3-propylthiophene, 4-ethylpyridine, and 4-butylquinoline; and compounds each having a aromatic ring fused to an nonaromatic ring, which nonaromatic ring has methylene group at an adjacent position to the aromatic ring, such as dihydronaphthalene, indene, indane, tetrahydronaphthalene, fluorene, acenaphthene, phenalene, indanone, and xanthene.

Exemplary nonaromatic compounds (A4-2 ) each having methyl group or methylene group at an adjacent position to an unsaturated bond include (A4-2a) chain unsaturated hydrocarbons each having methyl group or methylene group at an "allylic position"; and (A4-2b) compounds each having methyl group or methylene group at an adjacent position to carbonyl group.

Example of the chain unsaturated hydrocarbons (A4-2a) include chain unsaturated hydrocarbons having about 3 to about 20 carbon atoms, such as propylene, 1-butene, 2-butene, 1-pentene, 1-hexene, 2-hexene, 1,5-hexadiene, 1-octene, 3-octene, and undecatriene. Exemplary compounds (A4-2b) include ketones (e.g., chain ketones such as acetone, methyl ethyl ketone, 3-pentanone, and acetophenone; and cyclic ketones such as cyclohexanone) and carboxylic acids and derivatives thereof (e.g., acetic acid, propionic acid, butanoic acid, pentanoic acid, hexanoic acid, heptanoic acid, phenylacetic acid, malonic acid, succinic acid, glutaric acid, and esters of them).

The nonaromatic cyclic hydrocarbons (A5) include (A5-1) cycloalkanes; and (A5-2) cycloalkenes.

Exemplary cycloalkanes (A5-1) include compounds each having a three- to thirty-membered cycloalkane ring, such as cyclopropane, cyclobutane, cyclopentane, cyclohexane, cycloheptane, cyclooctane, cyclononane, cyclodecane, cyclododecane, cyclotetradecane, cyclohexadecane, cyclotetracosane, cyclotriacontane, and derivatives of them. Preferred cycloalkane rings include five- to thirty-membered cycloalkane rings, of which five- to twenty-membered cycloalkane rings are more preferred.

Exemplary cycloalkenes (A5-2) include compounds each having a three- to thirty-membered cycloalkene ring, such as cyclopropene, cyclobutene, cyclopentene, cyclooctene, cyclohexene, 1-methyl-cyclohexene, isophorone, cycloheptene, and cyclododecaene; cycloalkadienes such as cyclopentadiene, 1,3-cyclohexadiene, and 1,5-cyclooctadiene; cycloalkatrienes such as cyclooctatriene; and derivatives of them. Preferred cycloalkenes include compounds each having a three- to twenty-membered ring, of which compounds each having a three- to twelve-membered ring are more preferred.

The conjugated compounds (A6) include (A6-1) conjugated dienes; (A6-2) α,β-unsaturated nitriles; and (A6-3) α,β-unsaturated carboxylic acids and derivatives thereof (e.g., esters, amides, and acid anhydrides).

Exemplary conjugated dienes (A6-1) include butadiene, isoprene, 2-chlorobutadiene, and 2-ethylbutadiene. As used herein "conjugated dienes (A6-1)" also include compounds having a double bond conjugated with a triple bond, such as vinylacetylene.

Exemplary α,β-unsaturated nitriles (A6-2) include (meth)acrylonitriles. Exemplary α,β-unsaturated carboxylic acids and derivatives thereof (A6-3) include (meth)acrylic acids; (meth)acrylic esters such as methyl (meth)acrylates, ethyl (meth)acrylates, isopropyl (meth)acrylates, butyl (meth)acrylates, and 2-hydroxyethyl (meth)acrylates; and (meth)acrylamide derivatives such as (meth)acrylamides and N-methylol (meth)acrylamides.

Examples of the amines (A7) include primary or secondary amines including aliphatic amines such as methylamine, ethylamine, propylamine, butylamine, dimethylamine, diethylamine, dibutylamine, ethylenediamine, 1,4-butanediamine, hydroxylamine, and ethanolamine; alicyclic amines such as cyclopentylamine and cyclohexylamine; aromatic amines such as benzylamine and toluidine; and cyclic amines to which an aromatic or nonaromatic ring may be fused, such as pyrrolidine, piperidine, piperazine, and indoline.

Examples of the aromatic hydrocarbons (A8) include aromatic compounds having at least one benzene ring, such as benzene, naphthalene, acenaphthylene, phenanthrene, anthracene, and naphthacene, of which fused polycyclic aromatic compounds having at least two (e.g., two to ten) benzene rings being fused are preferred. Such aromatic hydrocarbons may have one or more substituents. Specific examples of aromatic hydrocarbons having one or more substituents include 2-chloronaphthalene, 2-methoxynaphthalene, 1-methylnaphthalene, 2-methylnaphthalene, 2-methylanthracene, 2-t-butylanthracene, 2-carboxyanthracene, 2-ethoxycarbonylanthracene, 2-cyanoanthracene, 2-nitroanthracene, and 2-methylpentalene. To the benzene ring(s), a nonaromatic carbon ring, aromatic heterocyclic ring, or nonaromatic heterocyclic ring may be fused.

Examples of the linear alkanes (A9) include linear alkanes having about 1 to about 30 carbon atoms (preferably having about 1 to about 20 carbon atoms), such as methane, ethane, propane, butane, pentane, hexane, heptane, octane, nonane, decane, dodecane, tetradecane, and hexadecane.

The olefins (A10) may be whichever of α-olefins and internal olefins, each of which may have one or more substituents (e.g., the aforementioned substituents such as hydroxyl group and acyloxy groups) and also include other olefins each having two or more carbon-carbon double bonds such as dienes. Exemplary olefins (A10) include chain olefins such as ethylene, propylene, 1-butene, 2-butene, isobutene, 1-hexene, 2-hexene, 1-acetoxy-3,7-di-methyl-2,6-octadiene, styrene, vinyltoluene, α-methylstyrene, 3-vinylpyridine, and 3-vinylthiophene; and cyclic olefins such as cyclopentene, cyclohexene, cycloheptene, cyclooctene, cyclodecene, cyclododecene, 1,4-cyclohexadiene, limonene, 1-p-menthene, 3-p-menthene, carveol, bicyclo[2.2.1]hept-2-ene, bicyclo[3.2.1]oct-2-ene, α-pinene, and 2-bornene.

Each of different compounds (A) capable of forming a free radical may be used alone or in combination, and in the latter case, the compounds used in combination may belong to the same or different categories. When two or more types of these compounds, especially two or more types of these compounds belonging to different categories, are used in a reaction, one of the substrates may act as a co-reacting agent (e.g., co-oxidizing agent) with respect to the other, and this may significantly improve the reaction rate.

Among the substrates, especially preferred substrates for use in the present invention are hydrocarbons including hydrocarbons each having methine carbon atom, such as adamantane and other bridged cyclic compounds having methine group; aromatic hydrocarbons each having methyl group or methylene group at an adjacent position to an aromatic ring, such as toluene and xylenes; and nonaromatic cyclic hydrocarbons such as cyclohexane and other cycloalkanes. According to the present invention, such hydrocarbons can industrially efficiently give, for example, hydroperoxides, alcohols, carbonyl compounds, and/or carboxylic acids in high yields.

Molecular oxygen can be used as oxygen acting as an oxidizing agent. The oxygen may be formed within the reaction system. The molecular oxygen is not especialy limited and can be whichever of pure oxygen; a diluted oxygen diluted with an inert gas such as nitrogen, helium, argon, or carbon dioxide gas; and air. Though suitably selectable according to the type of the substrate, the amount of the molecular oxygen is generally about 0.5 mole or more (e.g., 1 mole or more), preferably from about 1 to about 100 moles, and more preferably from about 2 to about 50 moles, per 1 mole of the substrate. The molecular oxygen is often used in excel moles to the substrate.

The oxidation reaction is carried out in the presence of, or in the absence of, a solvent. Exemplary solvents include organic acids such as acetic acid and propionic acid; nitriles such as acetonitrile, propionitrile, and benzonitrile; amides such as formamide, acetamide, dimethylformamide (DMF), and dimethylacetamide; aliphatic hydrocarbons such as hexane and octane; halogenated hydrocarbons such as chloroform, dichloromethane, dichloroethane, carbon tetrachloride, chlorobenzene, and trifluoromethylbenzene; nitro compounds such as nitrobenzene, nitromethane, and nitroethane; esters such as ethyl acetate and butyl acetate; and mixtures of these solvents.

The process according to the present invention allows a reaction to proceed smoothly under mild conditions. The reaction temperature can be appropriately selected according typically to the types of the substrate and the target compound and is, for example, from about 0°C to about 300°C, and preferably from about 20°C to about 200°C. The reaction can be performed under normal atmospheric pressure or under a pressure (under a load). When the reaction is performed under a pressure, the pressure is generally from about 0.1 to about 10 MPa, for example, from about 0.15 to about 8 MPa, and preferably from about 0.5 to about 8 MPa. The reaction time can be appropriately selected according to the temperature and pressure of reaction within ranges of, for example, from about 10 minutes to about 48 hours.

The reaction can be performed in the presence of, or under flow of, oxygen according to a common procedure or system such as a batch system, a semibatch system, or a continuous system. The reaction is preferably performed in a fluidized bed system or fixed bed system.

After the completion of the reaction, a reaction product can be separated and purified according to a separation procedure such as filtration, concentration, distillation, extraction, crystallization, recrystallization, or column chromatography, or any combination of them.

The process according to the present invention gives an oxidized product corresponding to the type of the substrate and the reaction conditions. Exemplary oxidized products include hydroperoxides, alcohols, carbonyl compounds (aldehydes or ketones), and carboxylic acids. Descriptions about such reaction products can be found typically in JAP-A No. H08-38909, JP-A No. H09-327626, JP-A No. H10-286467, and JP-A No. 2000-219650 (examples typically using a N-hydroxyphthalimide catalyst).

Typically, when the heteroatom-containing compound (A1) having a carbon-hydrogen bond at an adjacent position to the heteroatom is used as the substrate, the carbon atom at an adjacent position to the heteroatom is oxidized. For example, a primary alcohol gives a corresponding aldehyde or carboxylic acid; a secondary alcohol gives, for example, a corresponding ketone and/or carboxylic acid; a 1,3-diol gives a corresponding hydroxyketone; and a 1,2-diol gives a corresponding carboxylic acid through oxidative cleavage [see JP-A No. 2000-212116; and JP-A No. 2000-219652 (examples typically using a N-hydroxyphthalimide catalyst)]. An ether gives a corresponding ester or acid anhydride [see JP-A No. H10-316610 (examples typically using a N-hydroxyphthalimide catalyst)]. The process further allows a primary or secondary alcohol to give hydrogen peroxide [see PCT International Publication Number WO 00/46145 (examples typically using a N-hydroxyphthalimide catalyst)].

A compound (A2) having a carbon-heteroatom double bond, if used as the substrate, gives an oxidation reaction product according typically to the type of the heteroatom. For example, the oxidation of a ketones gives a corresponding ester, and further cleavage thereof gives, for example, a carboxylic acid. Typically, a cyclic ketone such as cyclohexanone gives a dicarboxylic acid such as adipic acid. A Baeyer-Villiger type reaction proceeds under mild conditions when, for example, a heteroatom-containing compound (A1) having a carbon-hydrogen bond at an adjacent position to the heteroatom, such as a secondary alcohol (e.g., benzhydrol), is used as a co-reacting agent (co-oxidizing agent). As a result, a cyclic ketone gives a corresponding lactone and/or dicarboxylic acid, and a chain ketone gives a corresponding ester and/or carboxylic acid, respectively in good yields [see PCT International Publication Number WO 99/50204 (examples typically using a N-hydroxyphthalimide catalyst)]. An aldehyde gives a corresponding carboxylic acid.

A compound (A3) having methine carbon atom, if used as the substrate, can give an alcohol derivative, whose methine carbon having an introduced hydroxyl group, in a high yield. Typically, the oxidation of a bridged cyclic hydrocarbon (A3-la), such as adamantane, gives, in a high selectivity, an alcohol derivative having an introduced hydroxyl group at a bridgehead position, such as 1-adamantanol, 1,3-adamantanediol, and/or 1,3,5-adamantanetriol. A chain compound (A3-2) having methine carbon atom, such as isobutane, can give a tertiary alcohol, such as t-butanol, in a high yield [see JP-A No. H10-310543 (examples typically using a N-hydroxyphthalimide catalyst)]. It can further gives a corresponding ketone and/or carboxylic acid.

A compound (A4) having a carbon-hydrogen bond at an adjacent position to an unsaturated bond, if used as the substrate, typically gives an alcohol, a carboxylic acid, an aldehyde, and/or a ketone through efficient oxidation of the adjacent position to an unsaturated bond. For example, a compound having methyl group at an adjacent position to an unsaturated bond gives a primary alcohol or carboxylic acid in a high yield [see JP-A No. H8-38909, JP-A No. H9-327626, and JP-A No. H11-106377 (examples typically using a N-hydroxyphthalimide catalyst)]. A compound having methylene group or methine group at an adjacent position to an unsaturated bond gives a secondary or tertiary alcohol, ketone, or carboxylic acid according to the reaction conditions in a good yield.

More specifically, an aromatic compound whose aromatic ring being bound to an alkyl group or lower-order oxidized group thereof (a hydroxyalkyl group, formyl group, a formylalkyl group, or an oxo-containing alkyl group) gives an aromatic carboxylic acid whose aromatic ring being bound to carboxyl group, through the oxidation of the alkyl group or lower-order oxidized group thereof. Typically, toluene, ethylbenzene, isopropylbenzene, benzaldehyde, or a mixture of them gives benzoic acid; p-xylene, p-isopropyltoluene, p-diisopropylbenzene, p-tolualdehyde, p-toluic acid, p-carboxybenzaldehyde, or a mixture of them gives terephthalic acid; pseudocumene, dimethylbenzaldehyde, dimethylbenzoic acid, or a mixture of them gives trimellitic acid; durene, trimethylbenzaldehyde, trimethylbenzoic acid, or a mixture of them gives pyromellitic acid; 3-methylquinoline, for example, gives 3-quinolinecarboxylic acid, respectively in good yields. β-Picolin gives nicotinic acid.

A compound having methylene group at an adjacent position to a carbon-carbon double bond, for example, gives a secondary alcohol or ketone, and/or a carboxylic acid. In this case, the use of a cobalt(II) salt of an acid having a pKa of 8.0 or less, such as cobalt(II) acetate or cobalt(II) nitrate, as the promoter, gives a corresponding conjugated unsaturated carbonyl compound having an oxo group introduced into the methylene carbon atom in a high yield. More specifically, valencene yields nootkatone in a high yield.

A nonaromatic cyclic hydrocarbon (A5), if used as the substrate, gives an alcohol or ketone whose ring-constituting carbon atom having an introduced hydroxy group or oxo group. Under some reaction conditions, the nonaromatic cyclic hydrocarbon (A5) gives a corresponding dicarboxylic acid through oxidative cleavage of the ring. Typically, cyclohexane gives cyclohexylhydroperoxide, cyclohexanol, cyclohexanone, or adipic acid in a high selectivity under appropriately selected conditions. A cycloalkane such as cyclohexane gives a bis(1-hydroxycycloalkyl)peroxide such as bis(1-hydroxycyclohexyl)peroxide [see Japanese Patent Application Number 2000-345824 (examples typically using a N-hydroxyphthalimide catalyst)]. The use of a strong acid as the promoter allows adamantane to give adamantanone in a good yield [see JP-A No. H10-309469 (examples typically using a N-hydroxyphthalimide catalyst)].

A conjugated compound (A6), if used as the substrate, gives a variety of compounds according to its structure. Typically, a conjugated diene gives, for example, an alkenediol, a ketone, and/or a carboxylic acid through oxidation. Specifically, butadiene gives, for example, 2-butene-1,4-diol and/or 1-butene-3,4-diol through oxidation. The oxidation of an α,β-unsaturated nitrile or an α,β-unsaturated carboxylic acid or a derivative thereof causes the selective oxidation of the α,β-unsaturated bonding site and gives a compound having a single bond derived from the unsaturated bond and having a group at the beta-position converted into formyl group or acetal group (when reacted in the presence of an alcohol) or into an acyloxy group (when reacted in the presence of a carboxylic acid). More specifically, the oxidation of acrylonitrile and methyl acrylate, for example, in the presence of methanol gives 3,3-dimethoxypropionitrile and methyl 3,3-dimethoxypropionate, respectively.

An amine (A7), if used as the substrate, typically gives a Schiff base and/or oxime. An aromatic compound (A8) gives a corresponding quinone in a good yield by the coexistence typically of a compound (A4) having a carbon-hydrogen bond at an adjacent position to an unsaturated bond (e.g., fluorene) as a co-reacting agent (co-oxidizing agent) [see JP-A No. H11-226416 and JP-A No. H11-228484 (examples typically using a N-hydroxyphthalimide catalyst)]. A linear alkane (A9) typically gives an alcohol, a ketone, and/or a carboxylic acid.

An olefin (A10), if used as the substrate, gives a corresponding epoxy compound [see JP-A No. H11-49764 and PCT International Publication Number WO 99/50204 (examples typically using a N-hydroxyphthalimide catalyst)]. In this case, an epoxidation reaction proceeds under mild condition to give a corresponding epoxide in a good yield by the coexistence as a co-reacting agent (co-oxidizing agent) of, for example, a heteroatom-containing compound (A1) having a carbon-hydrogen bond at an adjacent position to the heteroatom, such as a secondary alcohol, or a compound (A4) having a carbon-hydrogen bond at an adjacent position to an unsaturated bond. Additionally, a corresponding alcohol and/or carboxylic acid, for example, can be obtained.

A corresponding lactam is given by a reaction of at least one compound selected from cycloalkanes, cycloalkanols, and cycloalkanones with oxygen (B4-1) as an oxygen-containing reacting agent and ammonia in the presence of the immobilized cyclic imide catalyst [see Japanese Patent Application No. 2000-345823 (examples typically using a N-hydroxyphthalimide catalyst)]. More specifically, ε-caprolactam is given by a reaction of at least one compound selected from cyclohexane, cyclohexanol, and cyclohexanone with oxygen and ammonia in the presence of the catalyst.

Though not yet clarified in detail, a reaction mechanism in the process according to the present invention is supposed to be as follows. During the reaction, an oxidized active species [e.g., imido-N-oxy radical (>NO•)] is formed as in the cases using N-hydroxyphthalimide as a catalyst, the oxidized active species withdraws hydrogen from the substrate and allows the substrate to form a free radical; the resulting free radical reacts with oxygen and thereby yields a corresponding oxidized product. The free radical is formed, for example, at the carbon atom at an adjacent position to the heteroatom in the compound (A1), at the carbon atom relating to the carbon-heteroatom double bond in the compound (A2), at the methine carbon atom in the compound (A3), or at the carbon atom at an adjacent position to the unsaturated bond in the compound (A4).

### [Recovery and Regeneration of Immobilized Cyclic Imide Catalyst]

After the completion of the reaction, the immobilized cyclic imide catalyst, if used as a fluidized bed, can be easily recovered from the reaction mixture through a physical separation procedure such as filtration or centrifugal separation. The catalyst, if used as a fixed bed, can be recovered by detaching from the device.

The immobilized cyclic imide catalyst may be deactivated during some type of reactions under some reaction conditions. When the recovered immobilized catalyst is deactivated, its decreased catalytic activity can be re-activated or regenerated by the action of a hydroxylamine. The way to allow a hydroxylamine to act thereon is not specifically limited. Typically, the immobilized catalyst can be regenerated, for example, by dispersing the recovered immobilized catalyst into an organic base (e.g., pyridine or triethylamine); adding a salt (e.g., an inorganic salt, such as a hydrochloride or sulfate, or an organic salt) of hydroxylamine thereto; carrying out a reaction between them under mild reaction conditions of a temperature of about room temperature to about 120°C; and carrying out filtration, washing or rinsing, and drying after the reaction to complete the regeneration of the immobilized catalyst. The reaction between the recovered immobilized catalyst and the hydroxylamine may be carried out in a solvent such as toluene or tetrahydrofuran (THF) as appropriate. The salt of hydroxylamine may be used in the form of an aqueous solution. Thus, a regenerated immobilized catalyst having an N-hydroxy imide skeleton [an immobilized catalyst of Formula (1) in which X is an -OR group; and R is hydrogen atom] can be obtained.

The hydroxyl group of the resulting immobilized catalyst having an N-hydroxyimide skeleton may be protected by a protecting group R (R is as defined above) that will be easily deprotected, such as acetyl group, benzoyl group, or benzyl group. This protection is carried out by using a common reaction for introducing a protecting group. Alternatively, an immobilized catalyst having an N-substituted oxyimide skeleton [an immobilized catalyst of Formula (1) in which X is an -OR group and R is a hydroxyl-protecting group] can be obtained by using, instead of a hydroxylamine in the reaction, a hydroxylamine derivative having a deprotectable protecting group R (e.g., acetyl group, benzoyl group, or benzyl group) on its oxygen atom (H₂N-OR; R is as defined above) or a salt thereof (organic salt or inorganic salt).

### EXAMPLES

The present invention will be illustrated in further detail with reference to several examples below. It should be noted, however, that these examples are never construed to limit the scope of the present invention. Reaction products were analyzed typically by gas chromatography and high-performance liquid chromatography.

### EXAMPLE 1

An immobilized N-hydroxyphthalimide catalyst was prepared according to the following scheme: 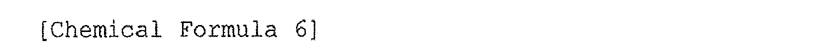 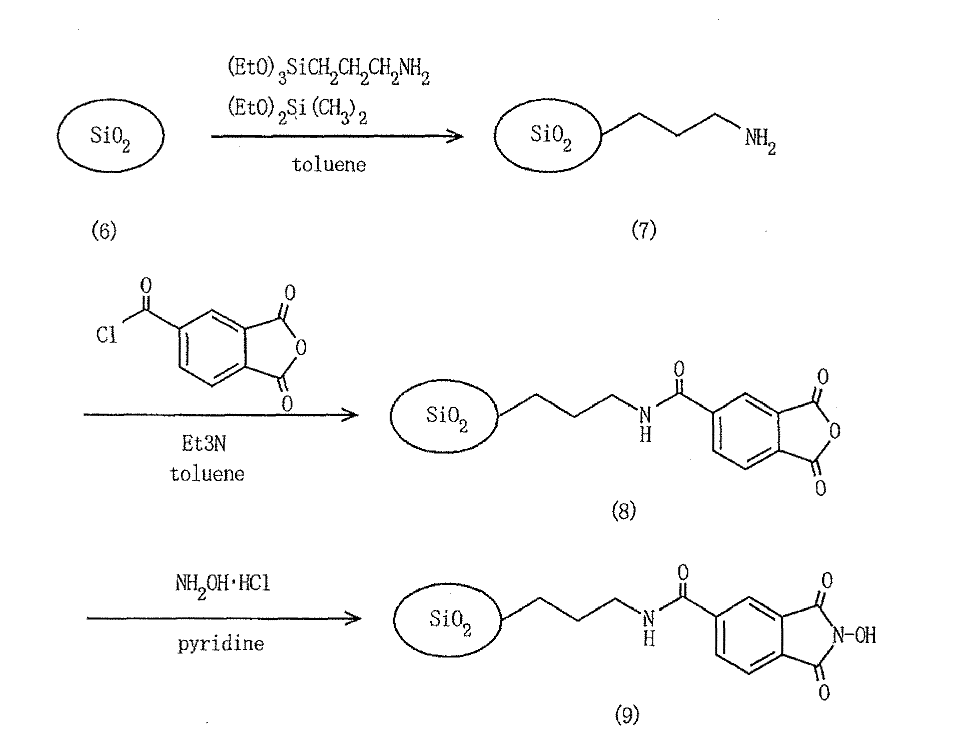 An aliquot (23 g) of a commercially available silica gel for column chromatography [Formula (6); supplied by Wako Pure Chemical Industries, Ltd., trade name "C-100"] was suspended in 50 mL of toluene, and to the thoroughly stirred suspension were added 0.883 g (4.0 mmol) of triethoxy(aminopropyl)silane and 6.0 g (40.5 mmol) of diethoxy(dimethyl)silane, followed by stirring under reflux for 14 hours. The treated silica gel was filtrated, washed with toluene several times, dried under reduced pressure, and thereby yielded an aminopropyl-immobilized silica gel [Formula (7)]. The amount of supported amino groups was found to be 0.105 mmol/g through titration.
In 10 mL of toluene was suspended 5 g (corresponding to 0.53 mmol of amino groups) of the above-prepared aminopropyl-immobilized silica gel [Formula (7)], and to the thoroughly stirred suspension were added 80.6 mg (0.8 mmol) of triethylamine and 113 mg (0.54 mmol) of anhydrous trimellitic chloride, followed by stirring at room temperature for a whole day and night. The treated aminopropyl-immobilized silica gel was filtrated, washed with toluene several times, dried under reduced pressure, and thereby yielded an immobilized N-hydroxyphthalimide precursor [Formula (8)]. The aminopropyl-immobilized silica gel represented by Formula (7) can also be a commercial product (e.g., one supplied by Sigma-Aldrich Inc.).
In 10 mL of pyridine was suspended 5 g of the above-prepared immobilized N-hydroxyphthalimide precursor [Formula (8)], and to the suspension was added 46 mg (0.66 mmol) of hydroxylamine hydrochloride, followed by a reaction under reflux for 4 hours. The treated immobilized N-hydroxyphthalimide precursor was collected by filtration, washed sequentially with toluene, THF, and diethyl ether, dried under reduced pressure, and thereby yielded 4.8 g of an immobilized N-hydroxyphthalimide catalyst [Formula (9)].

### EXAMPLE 2

An immobilized N-hydroxysuccinimide catalyst was prepared according to the following scheme: 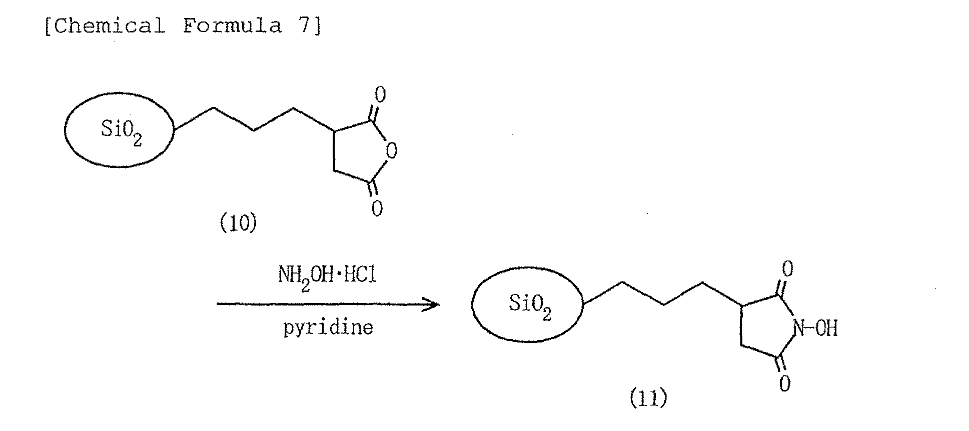 A commercially available succinic anhydride-supporting silica gel [Formula (10); supplied by Sigma-Aldrich Inc., supporting amount: 1.6 mmol/g] was used as an immobilized N-hydroxysuccinimide precursor. In 20 mL of pyridine was suspended 5.1 g (corresponding to 6.9 mmol of succinic anhydride) of the precursor, and to the thoroughly stirred suspension was added 544 mg (7.83 mmol) of hydroxylamine hydrochloride, followed by stirring at 110°C for 17 hours. The treated succinic anhydride-supporting silica gel was filtrated, washed sequentially with THF, 1 M diluted hydrochloric acid, THF, and diethyl ether, dried under reduced pressure, and thereby yielded 4.1 g of an immobilized N-hydroxysuccinimide catalyst [Formula (11)].

### EXAMPLE 3

In an autoclave equipped with a Teflon (registered trademark) inner cylinder, was prepared an acetic acid solution (3 mL) of 7.0 mg of manganese(II) acetate tetrahydrate, 6.6 mg of cobalt(II) acetate tetrahydrate, and 2.1 g (20 mmol) of p-xylene. To the solution was suspended 418 mg of an immobilized N-hydroxyphthalimide catalyst prepared according to the procedure of Example 1. The resulting mixture was stirred at 100°C under pressurized air (at 20 atmospheres, i.e., 2 MPa) for 15 hours. The reaction solution was analyzed to find that, in a conversion from p-xylene of 52%, there were produced p-methylbenzaldehyde in a selectivity of 12%, p-methylbenzoic acid in a selectivity of 58%, and terephthalic acid in a selectivity of 4%. After the completion of the reaction, the immobilized catalyst was separated by filtration, washed with ethyl acetate, dried under reduced pressure, and recovered as an immobilized catalyst. The recovered immobilized catalyst was reused in an oxidation reaction according to the same procedure, but the conversion was decreased to 26%, demonstrating that the activity of the catalyst was decreased.

### EXAMPLE 4

An oxidation reaction of p-xylene was performed a total of four times in the same manner as in Example 3 while using the immobilized N-hydroxyphthalimide catalyst prepared in Example 1 in each reaction (average of four reactions: conversion: 50%, selectivity: 12% for p-methylbenzaldehyde, 60% for p-methylbenzoic acid, and 2% for terephthalic acid), and the immobilized catalyst was recovered. The immobilized N-hydroxyphthalimide catalyst (1.88 g) whose activity had been decreased was suspended in 10 mL of pyridine, and 121.7 mg (1.75 mmol) of hydroxylamine hydrochloride was added to the suspension, followed by thoroughly stirring at 120°C for 16 hours. The treated catalyst was collected by filtration, washed sequentially with THF, 1 M diluted hydrochloric acid, THF, and diethyl ether, dried under reduced pressure, and thereby yielded 1.56 g of a regenerated immobilized N-hydroxyphthalimide catalyst.
In an autoclave equipped with a Teflon (registered trademark) inner cylinder, was prepared an acetic acid solution (3 mL) of 7.0 mg of manganese(II) acetate tetrahydrate, 6.6 mg of cobalt(II) acetate tetrahydrate, and 2.1 g (20 mmol) of p-xylene; and 427 mg of the above regenerated immobilized N-hydroxyphthalimide catalyst was suspended in the solution. The resulting mixture was stirred at 100°C under pressurized air (20 atmospheres, i.e., 2 MPa) for 15 hours. The reaction solution was analyzed to find that, in a conversion from p-xylene of 51%, there were produced p-methylbenzaldehyde in a selectivity of 8%, p-methylbenzoic acid in a selectivity of 57%, and terephthalic acid in a selectivity of 4%. This demonstrates that the catalyst was regenerated to have an activity equivalent to that immediately after preparation.

### EXAMPLE 5

In an autoclave equipped with a Teflon (registered trademark) inner cylinder was prepared an acetic acid solution (5 mL) of 7.3 mg of manganese(II) acetate tetrahydrate, 8.4 mg of cobalt(II) acetate tetrahydrate, and 221 mg (2 mmol) of p-xylene; and 134 mg of an immobilized N-hydroxysuccinimide catalyst prepared by the procedure of Example 2 was suspended in the solution. The resulting mixture was stirred at 100°C under pressurized air (at 20 atmospheres, i.e., 2 MPa) for 4 hours. The reaction solution was analyzed to find that, in a conversion from p-xylene of 97%, there were produced p-methylbenzaldehyde in a selectivity of 1%, p-methylbenzoic acid in a selectivity of 50%, and terephthalic acid in a selectivity of 20%. After the completion of the reaction, the products were dissolved in N,N-dimethylformamide (DMF), from which the immobilized catalyst was separated by filtration, washed with ethyl acetate, dried, and thereby recovered.

### EXAMPLE 6

In an autoclave equipped with a Teflon (registered trademark) inner cylinder, was prepared an acetic acid solution (3 mL) of 9.1 mg of manganese(II) acetate tetrahydrate, 7.0 mg of cobalt(II) acetate tetrahydrate, and 2.14 mg (20 mmol) of p-xylene; and 128 mg of an immobilized N-hydroxysuccinimide catalyst prepared by the procedure of Example 2 was suspended in the solution. The resulting mixture was stirred at 100°C under pressurized air (at 20 atmospheres , i.e., 2 MPa) for 4 hours. The reaction solution was analyzed to find that, in a conversion from p-xylene of 39%, there were produced p-methylbenzaldehyde in a selectivity of 20%, p-methylbenzoic acid in a selectivity of 44%, and terephthalic acid in a selectivity of 3%. After the completion of the reaction, the products were dissolved in N,N-dimethylformamide (DMF), from which the immobilized catalyst was separated by filtration, washed with ethyl acetate, dried, and thereby recovered. Industrial Applicability

According to the present invention, organic compounds such as hydrocarbons can be smoothly oxidized under mild conditions. The catalysts used herein, as being solid catalysts, can be easily separated from a reaction product and easily recovered from a reaction mixture. The catalysts can also be easily prepared, because the linkage group linking the cyclic imide skeleton with the inorganic support is a bivalent hydrocarbon group or a group composed of a bivalent hydrocarbon group and an amide bond. In addition, the catalysts are suitable as catalysts for oxidation reactions and can be used over a long period of time, because the linkage group has no factor inhibitory upon such oxidation reactions. The recovered catalysts, if deactivated, can be regenerated according to a simple procedure, and the regenerated catalysts are reusable.

## Claims

1. An immobilized cyclic imide catalyst, having a structure represented by following Formula (1): 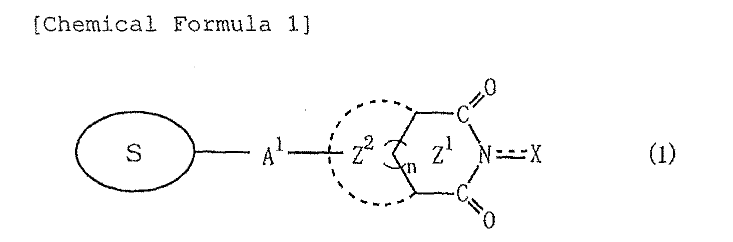 wherein X represents oxygen atom or an -OR group, wherein R represents hydrogen atom or a hydroxyl-protecting group; "n" represents 0 or 1; Z¹ represents a five- or six-membered cyclic imide skeleton to which an aromatic or nonaromatic ring Z² may be adjacent; elliptically shaped moiety "S" represents an inorganic support; A¹ represents a linkage group linking the inorganic support S with the cyclic imide skeleton Z¹ or with the ring Z² and is either a bivalent hydrocarbon group or a group composed of a bivalent hydrocarbon group and an amide bond (-NHCO-); the cyclic imide skeleton Z¹ and the ring Z² may each be substituted; and the structure may have, per molecule, two or more of the cyclic imide skeleton Z¹ to which the ring Z² may be adjacent.

2. The immobilized cyclic imide catalyst of claim 1, wherein, in Formula (1), (i) "n" is 0 and the ring Z² is a six-membered aromatic or nonaromatic carbon ring possessing one side in common with the cyclic imide skeleton Z¹, or (ii) "n" is 1 and the ring Z² is naphthalene ring or decahydronaphthalene ring possessing two sides in common with the cyclic imide skeleton Z¹.

3. The immobilized cyclic imide catalyst of claim 1, wherein the inorganic support S comprises at least one member selected from the group consisting of a silica, an alumina, a titania, a zirconia and a ceria, or comprises a conposite oxide of two or more elements selected from the group consisting of silicon, aluminum, zirconium and cerium.

4. A process for the oxidation of an organic compound, the process comprising the step of carrying out an oxidation reaction of the organic compound in the presence of the immobilized cyclic imide catalyst according to any one of claims 1 to 3.

5. The process for the oxidation of an organic compound of claim 4, wherein the oxidation reaction is carried out in a fluidized bed system or fixed bed system.

6. The process for the oxidation of an organic compound of claims 4 or 5, wherein a hydrocarbon is oxidized into at least one compound selected from the group consisting of a hydroperoxide, an alcohol, a carbonyl compound and a carboxylic acid.
